# EUROPEAN PATENT APPLICATION

(11) **EP 1 674 569 A1**
(43) Date of publication of application: **28.06.2006**
(21) Application number: 04788311.1
(22) Date of filing: 29.09.2004
(51) Int. Cl.: C12N 15/10, C12N 9/12, C12N 11/02, C12M 1/00, C12Q 1/68

(54) **SUPPORT HAVING ENZYME IMMOBILIZED THEREON, PRINT, REAGENT KIT, PROCESS FOR PRODUCING THE SUPPORT, METHOD OF PRESERVING ENZYME AND METHOD OF REVITALIZING ENZYME**

(30) Priority: 30.09.2003 JP 2003339542
(71) Applicant: RIKEN, Wako-shi, Saitama 351-0198 (JP); Kabushiki Kaisha Dnaform, Tokyo 108-0073 (JP)
(72) Inventor: HAYASHIZAKI, Yoshihide, Tsukuba-shi, Ibaraki 3050061 (JP); KAMIYA, Mamoru, c/o Kabushiki Kaisha Dnaform, Tokyo 1080073 (JP)
(74) Representative: Woods, Geoffrey Corlett
(86) International application number: PCT/JP2004/014245
(87) International publication number: WO 2005/030958

(57) **Abstract**

The invention enables enzymes to be stored in a simple manner. Disclosed are a support having an enzyme and a protecting agent for the enzyme fixed thereon; a printed material and a reagent kit comprising the support; a method for preparing the support; a method for restoring the enzyme fixed on the support; and a method for storing an enzyme in the form of being fixed on a support as a mixture with a protecting agent for the enzyme.

SEQUENCE LISTING

<110> RIKEN
      KABUSHIKI KAISHA DNAFORM

<120> Enzyme-fixing supports, printed materials, reagent
      kits, a method for preparing the support, a method for
      storing an enzyme and a method for renaturing an enzyme

<130> N96962 GCW

<140> EP 04788311.1
<141> 2004-09-29

<150> JP P2003-339542
<151> 2003-09-30

<160> 11

<170> PatentIn Ver. 2.1

<210> 1
<211> 18
<212> DNA
<213> Artificial Sequence

<220>
<223> Description of Artificial Sequence: synthetic DNA

<400> 1
tgtaaaacga cggccagt                                                18

<210> 2
<211> 24
<212> DNA
<213> Artificial Sequence

<220>
<223> Description of Artificial Sequence: synthetic DNA

<400> 2
agcggataac aatttcacac agga                                         24

<210> 3
<211> 1758
<212> DNA
<213> Mus musculus

<400> 3
cccggttctc tcccagagtc tgttccgctg tagaggtgac ctgactgctg gagactgcct 60
tttgcaggtg cagagatcgg ccttgcagtt tgcaataatg tctgaaccaa tcagagtcct 120
tgtgactgga gcagctggtc aaattgcata ttcactgttg tacagtattg gaaatggatc 180
tgtctttggg aaagaccagc ccatcattct tgtgctgttg gacatcaccc ccatgatggg 240
tgttctggac ggtgtcctga tggaactgca agactgtgcc cttccccttc tgcaggatgt 300
cattgcaacg gacaaagaag agattgcctt caaagacctg gatgtggctg tcctagtggg 360
ctccatgcca ataagggaag gcatggagag gaaggaccta ctgaaagcca atgtgaaaat 420
cttcaaatcc cagggcacag ccttggagaa atacgccaag aaatcagtta aggtcattgt 480
tgtgggaaac ccagccaata cgaactgcct gacagcctcc aagtcagcgc catcgatccc 540
caaggagaat ttcagttgcc tgactcgctt ggaccacaac cgagcaaaat ctcaaattgc 600
tcttaaactc ggtgtaaccg ctgatgatgt aaagaatgtc attatctggg gaaatcattc 660
atcgacccag tatccagatg tcaatcatgc caaggtgaaa ctgcaaggaa aggaagtcgg 720
tgtgtatgaa gccctgaaag acgacagctg gctgaaggga gagttcatca cgactgtgca 780
acagcgtggt gctgctgtca tcaaggctcg gaagctgtcc agtgcaatgt ctgctgcgaa 840
agccatcgca gaccacatca gagacatctg gtttggaacc ccagagggag agttcgtgtc 900
gatgggtgtt atctctgatg gcaactccta tggtgtccct gatgacctgc tctactcatt 960
ccctgtcgtg atcaagaata agacctggaa gtttgttgaa ggcctcccca ttaatgactt 1020
ctcccgtgaa aagatggacc tgacagcaaa ggagctgacc gaggaaaagg agaccgcttt 1080
tgagtttctc tcctctgcgt gactagacac tcgttttgac atcagcagac agccgaaggc 1140

tgaggaatca aaatgtcgtc tttgagccta gtaccaaaca gtaataatgc tacattcaaa 1200
ttgtgaacag caaaatattt taaatagtgt gtgctttatg atttgtgaaa gtctatcatg 1260
ttgttagtgc tgcaatctaa ataaaagtat attcaagtga aaatctctca gactctgttt 1320
ctactttata tttagtatct tcaggaaaac aagtttgccc aatagattat aattttactt 1380
ttttaattga ctaaaagaaa taaagatgga aaatattatg aagtaaagca ttagtctcta 1440
acataaacaa ggaagcccaa tcaatttcag agggatccca ttacttaagt ccttaaaggt 1500
tggttcatgt tttgctcata atttgatttt aaaattagct gtaagaaggt tgcagataat 1560
ctatcttctt tatattctat agcagaataa tgaagtcatt aatatttgat agccaataat 1620
accacactat taatatttgt aagctaagat tattagaaac ataaaactgt ttttgagtca 1680
gtctgttttc catgagaaga catgcatcat ctttgtgtgt tttgtgcatt actcagtgca 1740
ataaataacc ataatctc 1758

<210> 4
<211> 19
<212> DNA
<213> Artificial Sequence

<220>
<223> Description of Artificial Sequence: synthetic DNA

<400> 4
ccaggggctg ctgctgttg 19

<210> 5
<211> 18
<212> DNA
<213> Artificial Sequence

<220>
<223> Description of Artificial Sequence: synthetic DNA

<400> 5
catggtgggg cagtagcc 18

<210> 6
<211> 18
<212> DNA
<213> Artificial Sequence

<220>
<223> Description of Artificial Sequence: synthetic DNA

<400> 6
atgcgcgtgc tgcaggcg 18

<210> 7
<211> 23
<212> DNA
<213> Artificial Sequence

<220>
<223> Description of Artificial Sequence: synthetic DNA

<400> 7
tgcggattga gaagccttta ttg 23

<210> 8
<211> 27
<212> DNA
<213> Artificial Sequence

<220>
<223> Description of Artificial Sequence: synthetic DNA

<400> 8
gccggccaat gtacagtatt ggccggc 27

<210> 9
<211> 2438

<212> DNA
<213> Mus musculus

<400> 9
   gggtgttgcc gctgtcgccg cggtgaggga agtggacgcg atggccgggt ccgcgtgggt 60
   gtccaaggtc tctcggctgc tgggtgcatt ccacaacaca aaacaggtga caagaggttt 120
   tgctggtggt gttcagacag taactttaat tcctggagat ggaattggcc cagaaatttc 180
   agcctcagtc atgaagattt ttgatgctgg ccaaagcacc tattcagtgg gaggagcgca 240
   atgtcacagc aattcaagga ccaggaggaa agtgggatga tccctccaga agccaaggag 300
   tccatggata agaacaagat gggcttgaaa ggcccactaa agaccccaat agccgctggc 360
   catccatcta tgaatctgtt gcttcgtaag acatttgacc tttatgccaa tgtccggcca 420
   tgtgtctcaa ttgaaggtta taaaacccct tacacggatg taaatatcgt caccatccga 480
   gagaacacgg aaggagaata cagtggaatt gagcatgtga tcgttgatgg ggttgtgcag 540
   agcatcaagc tcatcaccga agaagcaagc aagcgcattg cagagtttgc cttcgagtac 600
   gctcggaaca accaccggag caacgtcaca gctgtgcaca aagctaacat catgaggatg 660
   tcagatgggc tctttctgca aaaatgcagg gaagttgcgg agaactgtaa agacattaaa 720
   tttaacgaga tgtaccttga tactgtatgt ttaaatatgg tacaagaccc atcccagttt 780
   gatgttcttg tcatgccaaa tttatacgga gacatcctta gtgatctgtg tgcaggactg 840
   attggaggtc ttggggtgac tccaagtggc aatattggag ccaacggtgt tgccatcttt 900
   gaatcggttc atggaacagc cccggacatt gcaggcaagg acatggccaa ccccacggcc 960
   ctcctgctta gtgctgtgat gatgcttcgc cacatgggac tttttgacca tgcagcaaaa 1020
   atcgaggctg catgttttgc tacaattaag gatggaaaga gcttaacaaa agatctggga 1080
   ggcaacgcga agtgctctga cttcacagaa gaaatctgtc gtagagtcaa agacttagat 1140
   tagcactcct gctggtggat ttgctgcagt cagtcaatca ctccaaaagg ataccctgta 1200
   atcctccttg agggcgccca ccattggttt gcttgcttct tgacagagta cgtttttgga 1260
   atctggcctt ttcttaacaa aacccttgca atggatgcac atgatggccc caggccttca 1320
   ttcaaagggt tttcccaagt gctggttgta tttattgtcc gtctggtaaa ccttattttg 1380
   taaactgtaa gtgaactgta tcatttatca ttgttaaccc attttacact tcaggcaaaa 1440
   tcattttcct caactgtaaa tattctgata cagaattaat aagagaagat atttaacttt 1500
   ttaacaaaag ccctggattt ttggtttatg aaaaacaaac tgggaataaa acagggtttc 1560
   aacaatcgca caagataaca ttattctaat actaatgggt acaaaagaaa tttactggga 1620
   aagttcacag caaaaaactg gtatatttct taaaaatatg gaaataaagt atttgtccta 1680
   tacatgaatt actattaata aaaatgtaag ctccaagaaa tccataatga atgatgtaat 1740
   tttgttacta catcggtaat ccttgtcaag gccccggatg ctctctgtgt atttgattct 1800
   ttggttacct tgagattcac tatttggggg gaagagcttt cagataaggg agatcactcc 1860
   tcactagaca gatcgtcagc attgcgagct gtcagccatg agagccagcc actgcagatc 1920
   ccctcccacg tggccacact ccagccagtg ctgcaggtga ccctggaaag gcctggctgc 1980
   cccttgactt tccctaaagc aaccagtcac tgccttctgc cccagtagca cccattacag 2040
   acttaattgc cgaggtggag ctgactcagc ccacgctcat acaaatcagg ccaagcgggg 2100
   gcctgtgtta ccagctgctg accatcaggt tctgcccctc attcttccca cagcctctgc 2160
   tccacagcat gaacctagcc tttggcccac accaaagcca agctgtcttc ccttagccct 2220
   tgcactagtt tgcaaactcg tggctttgca taatgtaccc tggtcccaag ggatttcttt 2280
   aacaacagat gtccctgtct gggtcatttt tttaaagctt ttatttggac ttacaatctt 2340
   ctgtgtattt tactttaaaa ctgctgcttt ccctgtctca ctggattgtt ctggttagca 2400
   gtggctttgg gttcacagta ataaagaact taagaact 2438

<210> 10
<211> 2160
<212> DNA
<213> Mus musculus

<400> 10
   ggatctaact ggggccggct tattacagct tgtgtgtacg cgcgggtgtg agccgggtta 60
   ttgaagtaaa aatgtccaga aaaatccaag gaggttctgt ggtggagatg caaggagatg 120
   aaatgacacg aatcatttgg gaattgatta aggaaaaact tattcttccc tatgtggaac 180
   tggatctgca tagctatgat ttaggcatag agaatcgtga tgccaccaat gaccaggtca 240
   ccaaagatgc tgcagaggct ataaagaaat acaacgtggg cgtcaagtgt gctaccatca 300
   cccccgatga gaagagggtt gaagaattca agttgaaaca aatgtggaaa tccccaaatg 360
   gcaccatccg aaacattctg ggtggcactg tcttcaggga agctattatc tgcaaaaata 420
   tcccccggct agtgacaggc tgggtaaaac ccatcatcat tggccgacat gcatatgggg 480
   accaatacag agcaactgat tttgttgttc ctgggcctgg aaaagtagag ataacctaca 540
   caccaaaaga tggaactcag aaggtgacat acatggtaca tgactttgaa gaaggtggtg 600
   gtgttgccat gggcatgtac aaccaggata agtcaattga agactttgca cacagttcct 660
   tccaaatggc tctgtccaag ggctggcctt tgtatctcag caccaagaac actattctga 720
   agaagtatga tgggcgtttc aaagacatct tccaggagat ctatgacaag aaatacaagt 780
   cccagtttga agctcagaag atctgctatg aacacaggct catagatgac atggtggccc 840
   aagctatgaa gtccgaggga ggcttcatct gggcctgtaa gaattacgat ggggatgtgc 900
   agtcagactc agtcgcccaa ggttatggct cccttggcat gatgaccagt gtgctgattt 960
   gtccagatgg taagacggta gaagcagagg ctgcccatgg cactgtcaca cgtcactacc 1020
   gcatgtacca gaaagggcaa gagacgtcca ccaaccccat tgcttccatt tttgcctggt 1080
   cccgagggtt agcccacaga gcaaagcttg ataacaatac tgagctcagc ttcttcgcaa 1140
   aggctttgga agacgtctgc attgagacca ttgaggctgg ctttatgact aaggacttgg 1200
   ctgcttgcat taaaggctta cccaatgtac aacgttctga ctacttgaat acatttgagt 1260
   ttatggacaa acttggagaa aacttgaagg ccaaattagc tcaggcccaa actttaaggt 1320
   caaacctggg cttagaatga gtctttgcgg taactaggtc cacaggttta cgtatttttt 1380
   tttttttttt tagtaacact caagattaaa aacaaaaatc attttgtaat tggtttagaa 1440
   gacaaagttg aacttttata tatgtttaca gtcttttttc tttttcatac agttattgcc 1500
   accttaatga atgtggtggg gaaatttttt taattgtatt ttattgtgta gtagcagtgt 1560
   aggaattatg ttagtacctg ttcacaatta actgtcatgt tttctcatgc tctaatgtaa 1620
   atgaccaaaa tcagaagtgc tccaagggtg aacaatagct acagtatggt tccccataag 1680
   gggaaaagag aaactcactt cccctgttgt ccatgagtgt gaacactggg gcctttgtac 1740
   gcaaatgttg tactgtgtgt gggagagcta tacagtaagc tcacataaga ctggaacaga 1800
   taggatgtgt gtagctaaaa tgcatggcag acgtgtttat aaagagcatg tatgtgtcca 1860
   atatactagt tatattttaa gaccactgga gaattccaag tctagaataa atgcagactg 1920
   gaggattctg ctctttgatt tctcttctcc tgtgacccag cctaagtatt atcctacccc 1980
   aagcagtaca tttcacccat gggcaataat gggagctgta ccgtttggat ttctgctgac 2040
   ctgctgcatt tcttttatat aaatgtgact tttttttccc agaagttgat attaaacact 2100
   attccagtct agtccttcta aactgttaat tttaattaaa atgaagtact aatgactctt 2160

<210> 11
<211> 3554
<212> DNA
<213> Mus musculus

<400> 11
gggggtggag ctgaacggga gacaggtact tgtggaaggc ttcaggacaa aatgtttcat 60
ttaaggactt gtgctgctaa gttaaggcca ttgacagcct cccagactgt taagacattt 120
tcacaaaaca aaccagcagc aattaggacg tttcaacaga ttcggtgcta ttctgcacct 180
gtagctgctg aaccatttct tagtgggact agttcgaact atgtggagga aatgtactgt 240
gcctggttgg agaatcccaa aagtgtacat aagtcatggg acattttttt ccgaaacacc 300
aatgctggag ccccaccggg cactgcctac cagagccccc tttccctgag tcgaagctcc 360
ctggctacca tggcccatgc acagtccctg gtggaagcac aacctaacgt cgacaaactc 420
gtggaggacc acttggcggt gcagtctctc atcagggcat atcagatacg agggcaccat 480
gtagcacagc tggacccccct ggggattttg gatgctgatc tggactcctc cgtgcccgct 540
gacattatct catccacaga caaacttggg ttctatggcc tacacgagtc tgaccttgac 600
aaggtcttcc acttacccac caccactttc atcgggggac aggagccagc acttcctctt 660
cgggagatca tccgtcggct ggagatggcc tactgccagc acattggtgt ggagttcatg 720
ttcattaatg atttggaaca atgccagtgg atccgacaga agtttgagac ccctggaatc 780
atgcagttca ccaatgagga gaagcggacc ttgctggcca ggcttgtacg atccaccagg 840
tttgaggagt tcctacagcg aaagtggtcc tcggagaagc gttttggtct ggaaggctgt 900
gaggtgctga tccctgccct caagacaatc attgatatgt caactcagat gaccctgaag 960
ctgtcatgta tgtatgcaag gtggcagctg agtggagaaa caccttccac aaggatgttg 1020
tagttgatct ggtgtgttat cgacgaaatg gccacaatga gatggacgaa cctatgttta 1080
cacagccact catgtacaag cagatccgca agcagaagcc tgtactgcag aagtatgcag 1140
aattgctagt ctcccagggt gtcgtcaatc agcctgagta cgaggaggaa atctccaagt 1200
atgataagat ctgtgaggaa gcatttacca gatccaaaga tgagaagatc ttgcacatca 1260
agcactggct ggattccccc tggcctggct tttcaccct ggatggacag cccaggagca 1320
tgacctgccc ctccactggc ctggaggagg atgtcttgtt ccacattgga aaggtggcca 1380
gctctgtacc tgtggagaac tttactatcc atggagggct gagccggatc ttgaagaccc 1440
gcagagagct tgtgacgaac cggactgtgg actgggccct ggcagagtac atggcatttg 1500
gctcactgct gaaggaaggc atccatgtgc ggctgagtgg ccaggatgtg gagcggggca 1560
ccttcagcca tcgccaccat gtgctccatg atcagaatgt tgacaaaaga acctgcatcc 1620
ccatgaacca cctttggcca aatcaggccc cttacactgt atgcaacagc tcgctgtctg 1680
agtacggtgt cctgggcttt gagctgggct ttgccatggc tagccctaat gctctggttc 1740
tctgggaggc ccagtttggt gacttcaaca acatggcaca gtgcatcatt gaccagttca 1800
tctgcccagg acaggcaaag tgggtgcggc agaatggcat tgtgctcctg ctgcctcatg 1860
gcatggaagg catgggtccc gagcattcct ctgaccgccc agagcggttt ctgcatatgt 1920
gcaatgatga cccatatgtc ctgcgtgact tgcaggaaga actctttgac atcaatcagc 1980
tatatgactg caactggatt gttgtcagct gttccacccg tggcaacttc ttccatgtgc 2040
tgcgacaaca gatcttgctg cccttccgta agccgttaat agtcttcact cccaaatccc 2100
tcttgcgcca ccgtgaggca agaactatct ttgacgatat gttgccagga acgcacttcc 2160
agcgtgtgat cccagaaaat ggacatgcag ctcaggaccc tcacaaagtc aagagacttc 2220
tcttctgcac tgggaaggtg tactatgacc tcacccgaga gcgcaaagcc aggaacatga 2280
aggaggaggt ggctattaca aggattgagc agctatcacc attccccttt gacctcctgt 2340
tgaaagaggc tcagaagtat cccaatgctg agctggcctg gtgccaggaa gagcacaaga 2400
accaaggcta ctatgactat gtcaagccaa gacttcgtac caccattgac cgtgctaagc 2460
ctgtctggta tgctgtccga gacccggcag ctgctccagc cactggcaac aagaaaacac 2520
acctgacaga gctgcagcgc tttctggaca cagcctttga cctggacgca ttcaagaaat 2580

tctcttagat gctcctggag ttgatgaggc catggccccc atgtccatga cgctctttgc 2640
ttctcaacta aagaatagtg cctcagcact gtccacacgt cccttcgctg tgccacacca 2700
cccctgttct cataggaatt aagttgtcca ctgcagtgct cagctgctcc ccggtcacat 2760
gctgcccagc ctgtgccgac ttctctcagg ctgcacaccg ttcatggaga ccggaaggag 2820
cagaataagg aaagggcccc tctcaggaca tcctagagaa ggaaggcagc tctggcccca 2880
cccatgcccc cagtgcaatc ctccagggta ggaacagaac cctatgtggc ttcccagggt 2940
actagcactc agccctcgtc acccatcaag tcgcagattc aaggccagga gtagtttcat 3000
cttgctaggg ccaagctgag agctcatgga ggaactatag ctgccaggat ttgggagtca 3060
tcaggatgtt gtgtgaatag agattgtcat ggggtattta gaggacttta gcagtgatgt 3120
tagtctagcc ctgctaccct tcttgggttt gggctgtatg tgggaaactt accccagcta 3180
ccacgcctgg agagcttggc tctgagtacg gcccagaagc tccattggct cccaacgcca 3240
ggcactgctg cctcttggtc ctgctgcctc tgctctcctg acccctcccc agtcacttca 3300
ttttctctgt tgttcccttg aacacacaga agctgttgac gaattctttt ttttgctgtg 3360
ccaaggcagg tcaaaagcag atcagtggat aagagcaagt tgtcccaagg agccagctgt 3420
ccttcctccc tcttttgacc tccactggga cacacctgat ttatttattt tggttaaaaa 3480
aaaaaaggaa atgaaaaaag aacaaccacc tttgcattgc atcggcttga cccataaact 3540
aagttatcat ggtc 3554

## Description

### Field of the Invention

The present invention relates to a support, a printed material and a reagent kit having an enzyme fixed thereon, a method for preparing the support, a method for storing an enzyme, and a method for restoring an enzyme.

### Background Art

Enzymes are proteins having catalytic activities and are involved in various biological reactions. Thus, enzymes contribute to the maintenance of life.

Enzymes are unstable at room temperature in the presence of moisture. Hence, enzymes are stored in a freeze-dried form or stored in a liquid containing a stabilizing agent at temperatures of -20°C or below.

Polymerase chain reaction (PCR) is a reaction for the amplification of a nucleic acid, which is catalyzed by an enzyme called "a DNA polymerase". A DNA polymerase is usually stored in a buffer at a temperature of -20°C. For this storage, a freezer is needed. When the enzyme is delivered from a supplier to a user, it is packaged in a container such as a container made of foamed polystyrene together with dry ice. These storage/delivery systems require specialized facilities and operations and, therefore, they are complicated and costly.

### Disclosure of the Invention

### Problems to be Resolved by the Invention

The object of the present invention is to provide a simple method for storing an enzyme.

### Means for Resolving the Problems

The present inventors have found that, when a DNA polymerase is fixed on a support as a mixture with trehalose for storage purpose and PCR is then performed using the support, the PCR reaction successfully proceeds. Based on this finding, the present invention has been accomplished.

The subject matters of the present invention are as follows.
(1) A support comprising an enzyme and a protecting agent for the enzyme fixed thereon.
(2) The support according to item 1, wherein the protecting agent is at least one chemical compound selected from the group consisting of trehalose and derivatives thereof.
(3) The support according to items 1 or 2, further comprising an enhancer for enzymatic reaction.
(4) The support according to any one of items 1 to 3, further comprising an aptamer for the enzyme thereon.
(5) A support comprising an enzyme and an aptamer for the enzyme fixed thereon.
(6) The support according to any one of items 1 to 5, wherein the enzyme is a DNA polymerase.
(7) The support according to item 6, further comprising a primer for the amplification of a nucleic acid of interest by a nucleic acid amplification reaction using the DNA polymerase.
(8) The support according to item 6, further comprising at least one member selected from the group consisting of a nucleic acid which serves as a template for the nucleic acid amplification reaction using the DNA polymerase, a primer for the amplification of the nucleic acid, and a buffer for the nucleic acid amplification reaction.
(9) A printed material comprising a support as recited in any one of items 1 to 8.
(10) A reagent kit comprising a support as recited in any one of items 1 to 8.
(11) A method for preparation of a support as recited in item 1, comprising: preparing a mixed solution of an enzyme and a protecting agent for the enzyme; applying the solution onto a support; and drying the support to fix a mixture of the enzyme and the protecting agent on the support.
(12) A method for restoration of an enzyme fixed on a support, comprising: immersing a support as recited in any one of items 1 to 8 in a liquid to leach out the enzyme into the liquid.
(13) A method for amplification of a nucleic acid, comprising: placing a support as recited in any one of items 6 to 8 in a liquid to leach out a DNA polymerase from the support; and performing a nucleic acid amplification reaction using the DNA polymerase.

The present invention also provides a method for storage of an enzyme in the form of being fixed on a support as a mixture with a protecting agent for the enzyme.

The present invention also provides a method for amplification of a nucleic acid, comprising: placing a support as recited in item (7) in a liquid to leach out a DNA polymerase and at least one member selected from the group consisting of a nucleic acid which serves as a template, primers for amplifying the nucleic acid and a buffer for the amplification reaction for the nucleic acid from the support; and performing the nucleic acid amplification reaction using the DNA polymerase and the nucleic acid which serves as a template and/or the primers.

Hereinbelow, the present invention will be described in detail.

The present invention provides a support having an enzyme and a protecting agent for the enzyme fixed thereon.

The enzyme may be any one as long as it has any catalytic activity. Examples of the enzyme include, but are not limited to, a DNA polymerase, an RNA polymerase, a reverse transcriptase, an RNase, a restriction enzyme, a methylase, a modifying enzyme, a ligase, a protease, a kinase, a phosphatase, a transferase, a glycosilase, a topoisomerase and a clonase.

The protecting agent may be any one as long as it can protect an enzyme from drying and store the enzyme stably. Examples of the protecting agent include trehalose and derivatives thereof, polysaccharides, PEG, dextran, Ficol, glycerol, surfactants, and PVA and derivatives thereof. Trehalose and derivatives thereof are particularly effective.

The protecting agent may be a commercially available product or may be synthesized according to any known method.

Trehalose is a non-reductive disaccharide composed of two 1,1-bonded D-glucose molecules and has three types of isomers: α,α-, α,β-and β,β-isomers, depending on the bonding manner.

Examples of the derivatives of trehalose include, but are not limited to, acid esters (e.g., fatty acid esters such as laureate, oleate, linoleate, linolenate, stearate, palmitate and myristate; carboxylates such as acetate and benzoate; and sulfate); alkyl ethers (e.g., ethers with C₈₋₂₅ alkyls); halides, nitrogen-containing derivatives and sulfur-containing derivatives of trehalose.

Trehalose and derivatives thereof are commercially available, but may be produced by any known method. The methods for production of trehalose and derivatives thereof can be found in, for example, "Developments in Food Carbohydrates", edited by C. K. Lee, issued by Applied Science Publishers, pp. 1-89, 1980; "Chemical and Pharmaceutical Bulletin", K. Yoshimoto et. al., vol. 30, No. 4, pp. 1,169-1,174, 1982; and Japanese Patent Application Laid-open No. 8-157491.

The protecting agent may be mixed with the enzyme in an amount of 10⁻⁵ to 10¹ M/U enzyme, preferably 10⁻⁴ to 10⁻¹ M/U enzyme.

On the support, an enhancer for enzymatic reaction may also be fixed. The enzymatic reaction enhancer may be any substance as long as it has an effect of enhancing the desired enzymatic reaction. The effect of enhancing the desired enzymatic reaction includes an effect of preventing the inhibition of the desired enzymatic reaction. Examples of the enzymatic reaction enhancer include, but are not limited to, sodium oxalate, potassium oxalate, sodium malonate, sodium maleate, dimethyl sulfoxide, betaines, glycerol, albumin, surfactants (e.g., Tween 20, Triton X100 and NP40), polyamines (e.g., ethylenediamine, trimethylenediamine, spermine, spermidine, diethylenetriamine, triethylenetetramine, tetraethylenepentamine, pentaethylenehexamine, 1,4-bis(3-aminopropyl)-piperazine, 1-(2-aminoethyl)piperazine, 1-(2-aminoethyl)piperidine, 1,4,10,13-tetraoxa-7,16-diazacyclooctadecane and tris(2-aminoethyl)amine), saccharides (e.g., glucose, fructose, galactose, maltose, sucrose, lactose and polysaccharides), sulfated polysaccharides and salts thereof (e.g., heparin, dextran sulfate), dithiothreitol, polyanions (e.g., DNA, RNA), polyhydric alcohols (e.g., aliphatic polyhydric alcohols such as ethylene glycol, propylene glycol, butanediol, hexanediol, octanediol, glycerin, sorbitan, trimethylol propane and neopentylglycol, diethylene glycol, triethylene glycol, polyethylene glycol and polypropylene glycol), ammonium sulfate and quaternary ammonium salts (e.g., hexadecyltrimethylammonium bromide, hexadecylpyridinium chloride, hexadimethrine bromide, hexafluorenium bromide and methylazolinium bromide). The enzymatic reaction enhancer includes, for example, Ampdirect® (Shimadzu Corporation), which is effective for enhancement of reactions with a DNA polymerase.

The enzymatic reaction enhancer may be fixed on the support in an appropriate amount. For example, a polyamine may be fixed on the support so that the polyamine is present in a reaction solution at a concentration of about 10 to 0.01 mM, preferably 2 to 0.5 mM. The enzymatic reaction enhancer may be fixed on the support in the same or different position as or from that of the mixture of the enzyme and the protecting agent.

The support may be any one as long as it can fix thereon the mixture of the enzyme and the protecting agent. Examples of the support include, but are not limited to, paper such as 60MDP paper (a product by Mishima Paper Co., Ltd., Japan), copy paper, woodfree paper, mechanical paper, kent paper, drawing paper, craft paper, paper for inkjet printing, tracing paper, Japanese paper, board paper, filter paper; glass substrates; silicon substrates; beads; column fillers; silica gel; nitrocellulose membrane; nylon membrane; and PVA membrane.

The support may have a thickness of 1 mm or less, for example. With a very small thickness (for example, about 0.1 mm), the workability of the support can be improved even if a number of enzyme- and protecting agent-fixed supports are stacked for distribution purposes, because the supports are not so bulky.

In addition to the enzyme and the protecting agent, the support may further comprise other components fixed thereon, such as a polynucleotide (e.g., DNA, RNA, a derivative or modified form thereof), an oligonucleotide (e.g., DNA, RNA, a derivative or modified form thereof), a protein (e.g., an antibody, a hormone), a polypeptide, an oligopeptide, a polysaccharide, an oligosaccharide, PNA, a low molecular compound (e.g., EDTA, a salt contained in a PCR buffer composition, Mg²⁺, a dNTP mixture) and a mixture thereof. The components other than the enzyme and the protecting agent may be fixed on the support in the same or different position as or from that of the mixture of the enzyme and the protecting agent. In particular, it is preferred that an aptamer for the enzyme be also fixed on the support. Accordingly, the present invention provides a support comprising an enzyme and an aptamer for the enzyme fixed thereon.

In one preferred embodiment of the present invention, in addition to the DNA polymerase and the protecting agent for the DNA polymerase, the support may further comprise primers for amplifying a nucleic acid of interest by nucleic acid amplification reaction using the DNA polymerase. The support of this type can be used in genotyping and identification of species. The support may further comprise an enhancer for the enzymatic reaction.

In another preferred embodiment of the present invention, in addition to the DNA polymerase and the protecting agent for the DNA polymerase, the support may further comprise at least one member selected from the group consisting of a nucleic acid which serves as a template for a nucleic acid amplification reaction (e.g., PCR) using the DNA polymerase, a primer for amplifying the nucleic acid, and a buffer for the nucleic acid amplification reaction. The support may also comprise an enhancer for enzymatic reaction.

For example, when a DNA polymerase is fixed on paper (support) for storage purpose, in addition to the DNA polymerase and the protecting agent, the paper may further have a set of primers (oligonucleotides), DNA which serves as a template for PCR reaction (which may be synthetic single- or double-stranded DNA or a vector having cDNA cloned therein), an aptamer for the DNA polymerase (functional RNA), components to be contained in a PCR reaction solution (i.e., Tris-HCl, KCl, MgCl, a dNTP mixture, etc.), EDTA and the like fixed thereon. In this case, fixation of these components may be achieved by any one of the following procedures: (1) the DNA polymerase, the protecting agent and the primer set are fixed together on the paper as a single spot, and the DNA as a template for PCR reaction, Tris-HCl and EDTA are fixed together on the paper as a separate single spot; (2) the DNA polymerase, the protecting agent, the primer set and, if required, an aptamer for the DNA polymerase are fixed together on the paper as a single spot; or (3) all of the components required for PCR reaction (i.e., DNA as a template for PCR reaction, the DNA polymerase, the primer set, Tris-HCl, KCl, MgCl, a dNTP mixture and the like and, if required, an aptamer for the DNA polymerase) together with the protecting agent are fixed on the paper as a single spot. For ease of distinction of the component (e.g., a DNA polymerase) spotted on paper, the component to be spotted may be mixed with a dye. Examples of the dye include, but are not limited to, cresol red, bromophenol blue and xylene cyanol.

The amount of the enzyme to be fixed on the support may be properly selected so that the desired enzymatic reaction can be achieved. For example, for PCR reaction, 5 ng or more of a DNA polymerase may be fixed per spot.

A support having a mixture of an enzyme and a protecting agent for the enzyme fixed thereon can be prepared in the following manner. First, a mixed solution of an enzyme and a protecting agent is prepared. The mixing ratio between the enzyme and the protecting agent is as described above. The solvent is preferably water. The mixed solution may further contain any one of the components other than the enzyme and the protecting agent as described above. Next, the mixed solution of the enzyme and the protecting agent is applied onto a support. For example, in the case where the support is paper, the mixed solution can be spotted on the paper using a syringe, a 96 pin-tool (Multi 96-multiblot replicator VP409, Bio Medical Science Inc., US), a disposable-type pin-tool or the like. The support is then dried to fix the mixture of the enzyme and the protecting agent thereon. Preferably, the support having the mixture of the enzyme and the protecting agent fixed thereon contains substantially no moisture.

By fixing an enzyme on a support as a mixture with a protecting agent for the enzyme as described above, the enzyme can be stored stably. As for the storage conditions, the support is preferably stored at room temperature under light shielding conditions while avoiding high humidities. In the case where the enzyme is a DNA polymerase, for example, when the DNA polymerase is stored at room temperature after being fixed on 60MDP paper as a mixture with trehalose, storage life of at least 6.5 months has been confirmed (the storage test is now being carried on).

For restoration of an enzyme fixed on a support as a mixture with a protecting agent for the enzyme as described above, the support having the mixture of the enzyme and the protecting agent fixed thereon may be immersed in a liquid to leach out the enzyme into the liquid. The liquid to be used for the immersion of the support may be any one as long as it enables the restoration of the enzyme. Examples of the liquid includes, but are not limited to, water and an aqueous solution containing an ingredient other than water. In the case where the enzyme fixed on a support is a DNA polymerase, for example, the liquid to be used for the immersion of the support is preferably water, a PCR reaction solution (i.e., an aqueous solution containing Tris-HCl, KCl, MgCl, a dNTP mixture and the like) or the like. The immersion may be performed at room temperature under atmospheric pressure for 1 to 3 minutes.

When a DNA polymerase is fixed on a support, the support may be placed in a liquid to leach out the DNA polymerase from the support and nucleic acid amplification reaction may be then performed using the leached-out DNA polymerase to amplify a nucleic acid.

The present invention also provides a printed material comprising a support having an enzyme and a protecting agent for the enzyme fixed thereon.

The printed material includes, but is not limited to, complete books (e.g., textbooks), handbooks, catalogues, journals, magazines, articles, , booklets, minibooklets, leaflets, pamphlets, reports, posters, cards and labels.

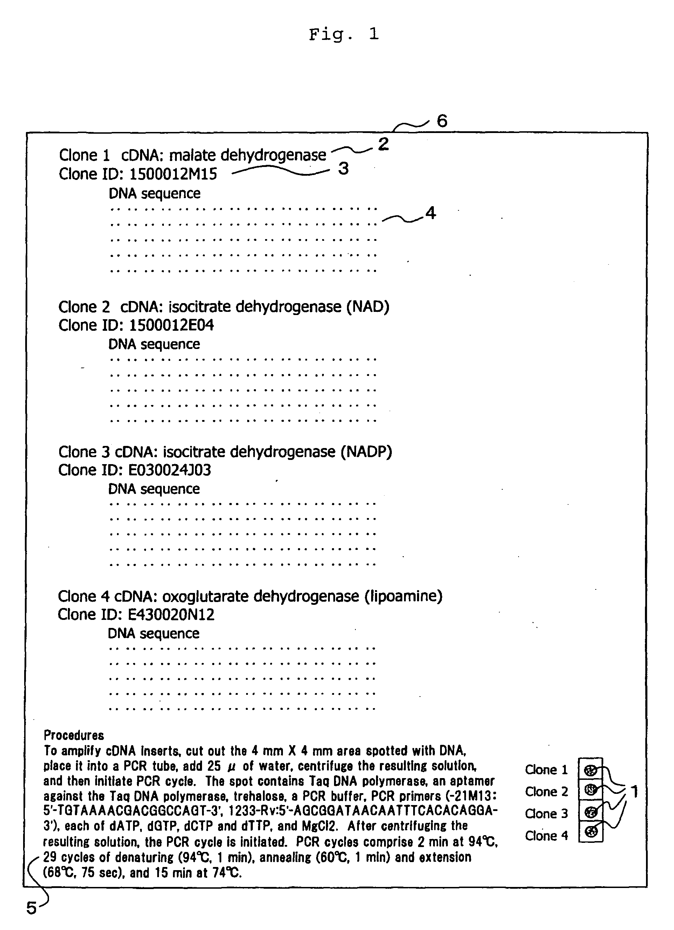

Fig. 1 shows an embodiment of the printed material according to the present invention, in which a mixture of an enzyme (a DNA polymerase) and trehalose together with a primer set, a cDNA clone which serves as a template for PCR reaction and other components required for the PCR reaction (i.e., Tris-HCl, KCl, MgCl, each dNTP and, if required, an aptamer for the DNA polymerase) is spotted on paper (support). The DNA polymerase and trehalose are spotted on the paper 6 (hereinafter, this spot is referred to as "DNA Spot 1"). In the paper 6, in addition to DNA spot 1, the name 2 of a protein encoded by the cDNA clone ("malate dehydrogenase"), the identification number 3 of the cDNA clone ("Clone ID"), the nucleotide sequence 4 of the cDNA clone ("DNA sequence") and the instructions 5 of the procedure for an experiment (PCR reaction) ("Procedures") are printed on the paper.

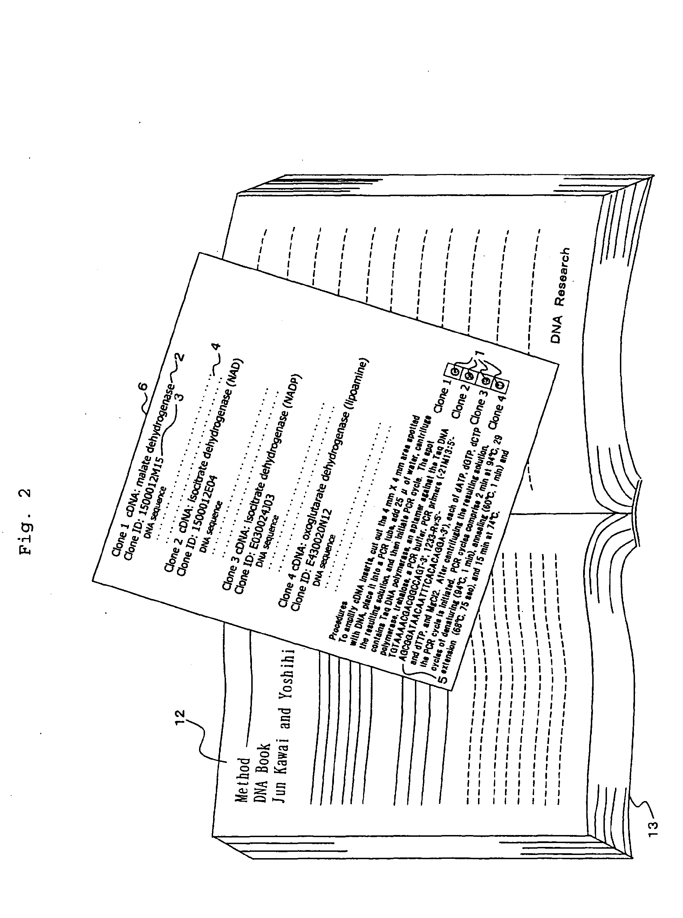

Fig. 2 shows a magazine 13 including a scientific article 12, to which the paper 6 having DNA spot 1 thereon shown in Fig. 1 is added as an appendix.

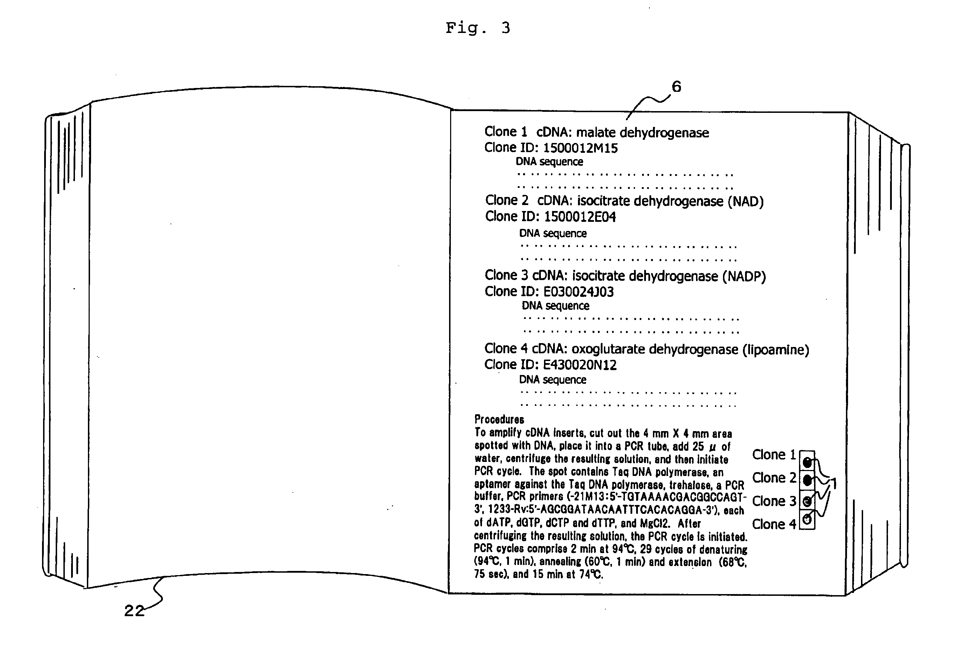

Fig. 3 shows a book 22 in which the paper 6 having DNA spot 1 thereon shown in Fig. 1 is bounded. This book may further include a table of contents.

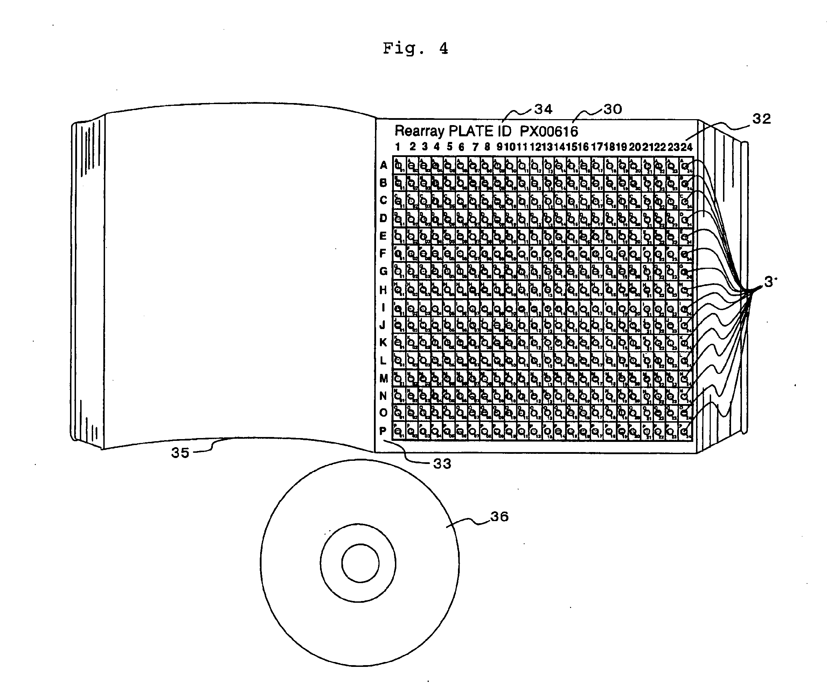

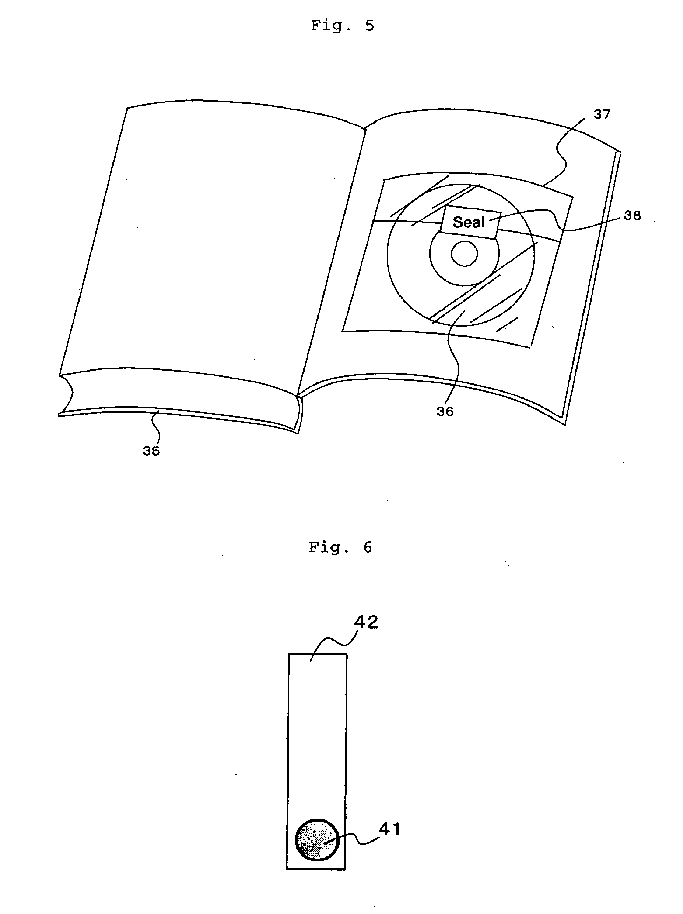

Fig. 4 shows another embodiment of the book in which paper (support) comprising a mixture of an enzyme (a DNA polymerase) and trehalose spotted thereon is bounded. On each grid of a checkerboard pattern of page 34 on which a mixture of an enzyme (a DNA polymerase) and trehalose are spotted, the DNA polymerase and trehalose together with a primer set, a cDNA clone which serves as a template for PCR reaction and other components required for the PCR reaction (i.e., Tris-HCl, KCl, MgCl, each dNTP and, if required, an aptamer for the DNA polymerase) are spotted (hereinafter, this spot is referred to as "DNA spot 31"). On the page 34 having these spots 31, symbols 32 (column number) and 33 (row number) for identification of each spot are printed. Also printed are the identification number 30 ("Rearray PLATE ID") of a page having DNA spotted thereon. The information about the spotted cDNA clone, such as EC number of an enzyme encoded by the cDNA clone, name of an enzyme encoded by the cDNA clone ("Gene name"), ID number ("RIKEN Clone ID") and deposition number ("Accession Number") of the clone, length of the cDNA clone insert ("cDNA Insert), length of a PCR reaction product ("After PCR") and the explanation of a reaction in which the enzyme encoded by the cDNA clone is involved, and the information about the primer set, such as nucleotide sequence of the primers, are recorded in a CD-ROM 36 (as a substitute for the CD-ROM, other recording medium such as FD and MD may be used). The recording medium is added as an appendix to the book (Fig. 5). In Fig. 5, the CD-ROM 36 is packed in a bag 37 and attached to the book 35 with the bag 37 being sealed with a seal 38. This book may also have a page on which a table of contents, an instruction manual for the spots containing the cDNA clone and primer set, and an access guide to the information recorded in the recording medium are printed.

Exemplary types of the printed material include the following: 1) an encyclopedia-like all-inclusive type (e.g., FANTOM clone, human metabolome); 2) a volume-separated type by item (e.g., function, organ) ; 3) a small volume type by subdivided subjects or contents which consists of one to several pages (e.g., loose-leaf type); and 4) a card type which is intended to have a smaller number of attachments.

The present invention also provides a reagent kit comprising a support having an enzyme and a protecting agent for the enzyme fixed thereon.

The reagent kit according to the present invention can be used as a nucleic acid amplification reaction (e.g., PCR) kit, a protein production kit, an antibody kit and other types of kits for use in a variety of experiments, tests, diagnoses and the like.

The reagent kit according to the present invention may be in the form of a printed material as described above. Other embodiments of the reagent kit are shown in Figs. 6 to 9.

Fig. 6 shows an embodiment of the reagent kit according to the present invention, in which a mixture of an enzyme (a DNA polymerase) and trehalose together with a primer set and other components required for PCR reaction (i.e., Tris-HCl, KCl, MgCl, each dNTP and, if required, an aptamer for the DNA polymerase) is spotted on paper (support). In the paper, the DNA polymerase and trehalose are spotted in a proper position on the paper (hereinafter, this spot is referred to as "DNA spot 41").

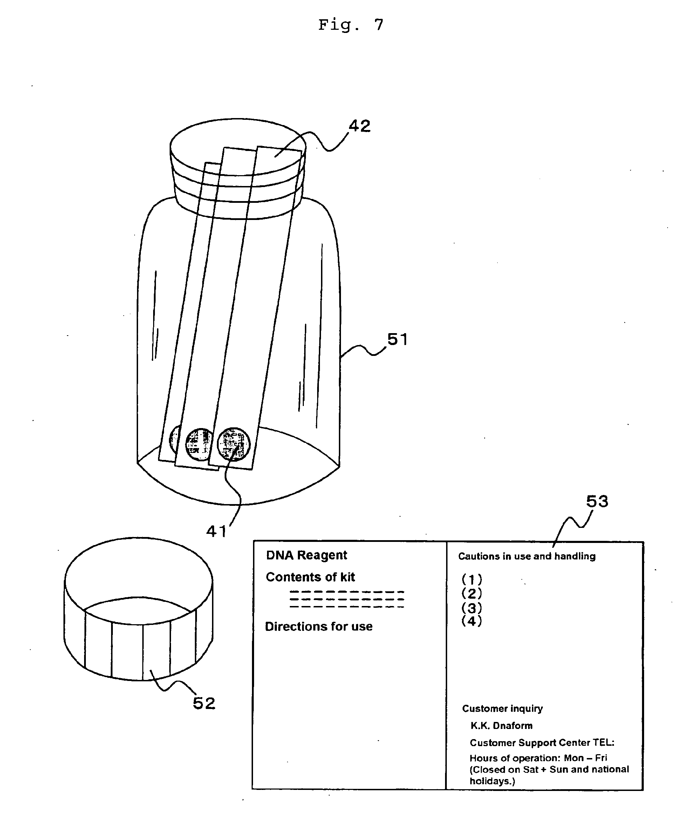

Fig. 7 shows an embodiment of the reagent kit, which comprises a sheet of paper 42 having a DNA spot 41 shown in Fig. 6 thereon. The paper 42 having the DNA spot 41 is placed in a light-proof bottle 51, and then stored or distributed with the bottle 51 hermetically sealed with a cap 52. The reagent kit may further include an instruction manual 53 on which the information about the kit, such as the contents of the kit (e.g., names/quantities of components included in the kit, recommended use, directions for storage/expiration date, package unit), directions for use, cautions in use and handling, and customer inquiry, is printed. The instruction manual 53 may be included in the light-proof bottle 51 or placed in a package box (now shown) including the light-proof bottle 51. Alternatively, the instruction manual may be printed on a label and attached on the light-proof bottle 51.

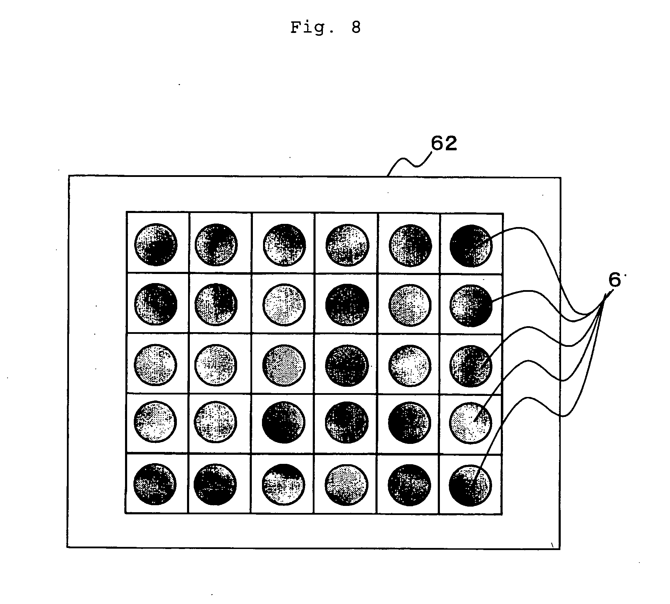

Fig. 8 shows an embodiment of the regent kit according to the present invention, in which a mixture of an enzyme (a DNA polymerase) and trehalose is spotted on paper (support). In the paper 62, the DNA polymerase and trehalose together with a primer set and other components required for PCR reaction (i.e., Tris-HCl, KCl, MgCl, each dNTP and, if required, an aptamer for the DNA polymerase) are spotted in a proper position on the paper (hereinafter, this spot is referred to as "DNA spot 61").

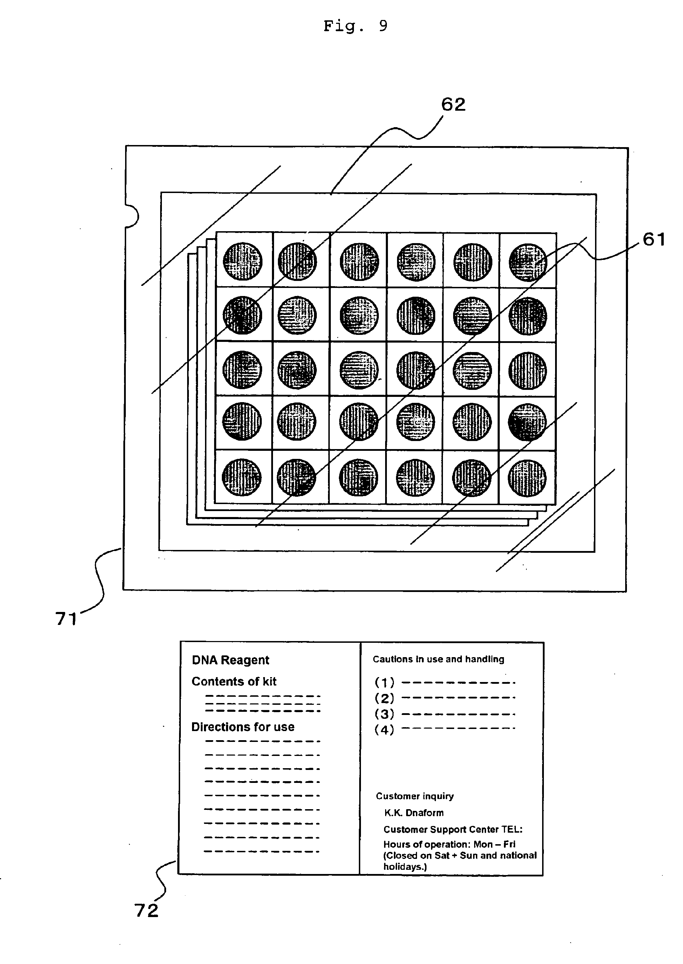

Fig. 9 shows an embodiment of the reagent kit comprising a sheet of paper 62 having DNA spots 61 thereon. The paper 62 having DNA spots 61 is placed in a packaging pack 71 and stored or distributed with the packaging pack 71 being hermetically sealed. The reagent kit may further include an instruction manual 72 on which the information about the kit, such as the contents of the kit (e.g., names/quantities of components included in the kit, recommended use, directions for storage/expiration date, package unit), directions for use, cautions in use and handling, and customer inquiry, is printed. The instruction manual 72 may be included in the packaging pack 71 or placed in a package box (now shown) including the packaging pack 71. Alternatively, the instruction manual 72 may be printed on a label and attached on the packaging pack 71 or the package box.

Hereinabove, the present invention is described with reference to several embodiments of the combination of a DNA polymerase and DNA. However, the invention is not limited to these embodiments and applicable to a variety of enzymes.

In this specification, it is noted that the mark "-" means a numerical range in which the numerical numbers given before and after the mark are included in the range as the minimum value and the maximum value, respectively.

### Effect of the Invention

According to the present invention, a simple method for storing an enzyme is provided.

This specification includes part or all of the contents as disclosed in the specification and/or drawings of Japanese Patent Application No. 2003-339542 based on which the present application claims priority.

### Brief Description of Drawings

Fig. 1 shows an embodiment of paper (support) comprising a mixture of a DNA polymerase and trehalose together with DNA (cDNA, primer, aptamer, etc.) spotted thereon.
Fig. 2 shows an embodiment of a magazine including a scientific article, in which paper comprising a mixture of a DNA polymerase and trehalose together with DNA (cDNA, primer, aptamer, etc.) spotted thereon is added as an appendix.
Fig. 3 shows an embodiment of a book in which paper comprising a mixture of a DNA polymerase and trehalose together with DNA (cDNA, primer, aptamer, etc.) spotted thereon is bounded.
Fig. 4 shows another embodiment of a book in which paper (support) comprising a mixture of a DNA polymerase and trehalose together with DNA (cDNA, primer, aptamer, etc.) spotted thereon is bounded.
Fig. 5 shows an embodiment of packaging into a bag a CD-ROM on which the information about cDNA spotted on the paper shown in Fig. 4 is recorded, with the CD-ROM being added as an appendix to the book with the bag sealed with a seal.
Fig. 6 shows an embodiment of paper (support) comprising a mixture of a DNA polymerase and trehalose together with DNA (cDNA, primer, aptamer, etc.) spotted thereon.
Fig. 7 shows an embodiment of a reagent kit comprising the paper shown in Fig. 6.
Fig. 8 shows an embodiment of paper (support) comprising a mixture of a DNA polymerase and trehalose together with DNA (cDNA, primer, aptamer, etc.) spotted thereon.
Fig. 9 shows an embodiment of a reagent kit comprising the paper shown in Fig. 8.
Fig. 10 shows the constitution of pFLC vector in which murine malate dehydrogenase cDNA is cloned.
Fig. 11 shows 60MDP paper having both a spot of a solution of murine malate dehydrogenase cDNA and a spot of a [polymerase + primer] solution.
Fig. 12 shows the electrophoretic results of a product of the PCR reaction performed using the spots shown in Fig. 11.
Fig. 13 shows 60MDP paper having a spot of a [primer + aptamer + polymerase] solution.
Fig. 14 shows the electrophoretic results of a product of the PCR reaction performed using the spot shown in Fig. 13.
Fig. 15 shows 60MDP paper having spots of a [cDNA + primer + aptamer + polymerase + PCR buffer composition] solution, wherein the cDNA is that for any one of murine malate dehydrogenase, murine isocitrate dehydrogenase (NADP), murine isocitrate dehydrogenase (NAD) and murine oxoglutarate dehydrogenase.
Fig. 16 shows the electrophoretic results of a product of the PCR reaction performed using the spots shown in Fig. 15.
Fig. 17 shows the electrophoretic results of a product of the PCR reaction performed using the spots of a [cDNA + primer + reaction enhancer (spermidine) + polymerase + PCR buffer composition] solution.

### Explanation of Reference Signs in the Drawings

1: DNA spots
2: Name of protein (enzyme such as malate dehydrogenase) encoded by cDNA clone
3: Identification number of cDNA clone
4: Nucleotide sequence of cDNA clone
5: Instruction manual for experimental procedure
6: Paper having a mixture of DNA polymerase and trehalose together with DNA (cDNA, primer, aptamer, etc.) spotted thereon
12: Scientific article
13: Magazine
22: Book
30: Identification number of page on which DNA is spotted
31: DNA spots
32: Symbol 1 for identification of spot (column number)
33: Symbol 2 for identification of spot (row number)
34: Page having a mixture of DNA polymerase and trehalose together with DNA (cDNA, primer, aptamer, etc.) spotted thereon
35: Book
36 CD-ROM
37: Bag
38: Seal
41: DNA spot
42: Paper (support)
51: Light-proof bottle
52: Cap
53: Instruction manual
61: DNA spots
62: Paper (support)
71: Packaging pack
72: Instruction manual

### Best Modes for Carrying out the Invention

Hereinbelow, the present invention will be described in great detail with reference to the following examples. However, the examples are illustrative only and the scope of the invention is not limited to these examples.

### Examples

### [Example 1]

DMA book having spots of cDNA clone and polymerase thereon

### <Synthesis of primers>

A set of primers having the following sequences were synthesized according to a conventional method.
Primer set 1:
-21M13: 5'-TGTAAAACGACGGCCAGT-3' (SEQ ID NO:1)
1233-Rv: 5'-AGCGGATAACAATTTCACACAGGA-3' (SEQ ID NO:2)

### <Preparation of cDNA solution>

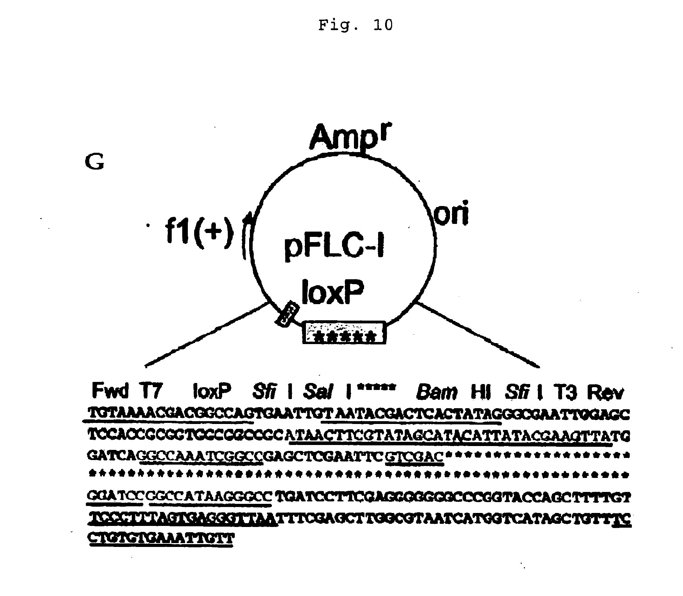

pFLC vector, into which murine malate dehydrogenase cDNA (Clone ID: 1500012M15,1758 bp) having the following nucleotide sequence collected from the Riken clones (http://fantom.gsc.riken.go.jp/) had been cloned (Fig. 10), was dissolved in TE buffer (10 mM Tris-HCl, pH 8.0, 1 mM EDTA) to give a concentration of 0.1 µg/µl.

### Murine malate dehydrogenase 1500012M15

1 cccggttctc tcccagagtc tgttccgctg tagaggtgac ctgactgctg gagactgcct 61 tttgcaggtg cagagatcgg ccttgcagtt tgcaataatg tctgaaccaa tcagagtcct
121 tgtgactgga gcagctggtc aaattgcata ttcactgttg tacagtattg gaaatggatc
181 tgtctttggg aaagaccagc ccatcattct tgtgctgttg gacatcaccc ccatgatggg
241 tgttctggac ggtgtcctga tggaactgca agactgtgcc cttccccttc tgcaggatgt
301 cattgcaacg gacaaagaag agattgcctt caaagacctg gatgtggctg tcctagtggg
361 ctccatgcca ataagggaag gcatggagag gaaggaccta ctgaaagcca atgtgaaaat
421 cttcaaatcc cagggcacag ccttggagaa atacgccaag aaatcagtta aggtcattgt
481 tgtgggaaac ccagccaata cgaactgcct gacagcctcc aagtcagcgc catcgatccc
541 caaggagaat ttcagttgcc tgactcgctt ggaccacaac cgagcaaaat ctcaaattgc
601 tcttaaactc ggtgtaaccg ctgatgatgt aaagaatgtc attatctggg gaaatcattc.
661 atcgacccag tatccagatg tcaatcatgc caaggtgaaa ctgcaaggaa aggaagtcgg
721 tgtgtatgaa gccctgaaag acgacagctg gctgaaggga gagttcatca cgactgtgca
781 acagcgtggt gctgctgtca tcaaggctcg gaagctgtcc agtgcaatgt ctgctgcgaa
841 agccatcgca gaccacatca gagacatctg gtttggaacc ccagagggag agttcgtgtc
901 gatgggtgtt atctctgatg gcaactccta tggtgtccct gatgacctgc tctactcatt
961 ccctgtcgtg atcaagaata agacctggaa gtttgttgaa ggcctcccca ttaatgactt
1021 ctcccgtgaa aagatggacc tgacagcaaa ggagctgacc gaggaaaagg agaccgcttt
1081 tgagtttctc tcctctgcgt gactagacac tcgttttgac atcagcagac agccgaaggc
1141 tgaggaatca aaatgtcgtc tttgagccta gtaccaaaca gtaataatgc tacattcaaa
1201 ttgtgaacag caaaatattt taaatagtgt gtgctttatg atttgtgaaa gtctatcatg
1261 ttgttagtgc tgcaatctaa ataaaagtat attcaagtga aaatctctca gactctgttt
1321 ctactttata tttagtatct tcaggaaaac aagtttgccc aatagattat aattttactt
1381 ttttaattga ctaaaagaaa taaagatgga aaatattatg aagtaaagca ttagtctcta
1441 acataaacaa ggaagcccaa tcaatttcag agggatccca ttacttaagt ccttaaaggt
1501 tggttcatgt tttgctcata atttgatttt aaaattagct gtaagaaggt tgcagataat
1561 ctatcttctt tatattctat agcagaataa tgaagtcatt aatatttgat agccaataat
1621 accacactat taatatttgt aagctaagat tattagaaac ataaaactgt ttttgagtca
1681 gtctgttttc catgagaaga catgcatcat ctttgtgtgt tttgtgcatt actcagtgca
1741 ataaataacc ataatctc (SEQ ID NO:3)

### <Preparation of [polymerase + primer] solution>

KOD plus polymerase (Toyobo Engineering Co., Ltd., Japan), trehalose and the primer set 1 were mixed together to prepare a solution having the final concentrations of 25 U/µl of KOD plus DNA polymerase, 0.1 M of trehalose and 2 µM of primer set 1.

### <Spotting of DNA>

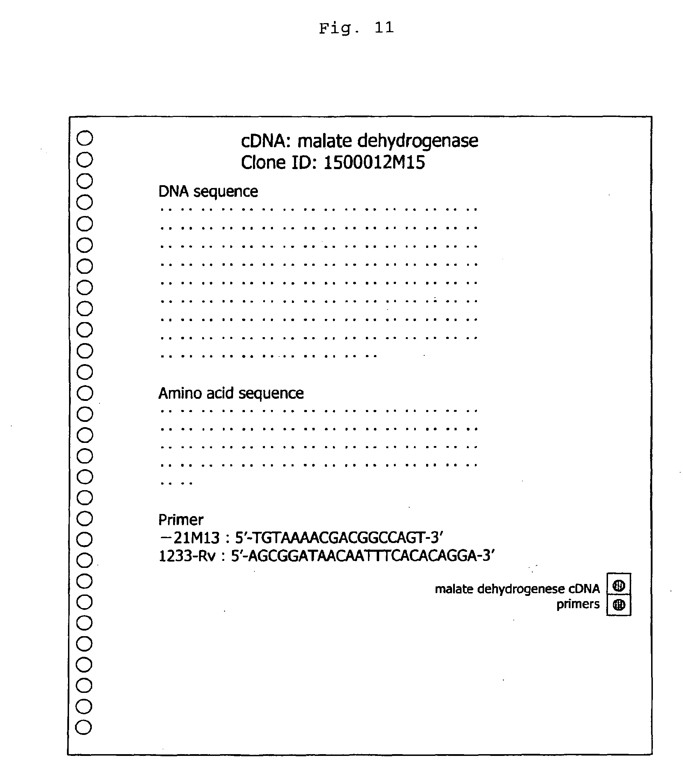

The cDNA solution and the [polymerase + primer] solution were spotted on a sheet of 60MDP paper (Mishima Paper Co., Ltd., Japan) using a 96 pin-tool (Multi 96-multiblot replicator VP409, Bio Medical Science Inc., US) so that the positions and the types of the spots could be identified, as shown in Fig. 11. The cDNA solution and the [polymerase + primer] solution were spotted at rates of 0.5 µl/spot and 1 µl/spot, respectively.

### <Recovery and amplification of DNA>

The spotted paper was dried at room temperature for at least 30 minutes. Thereafter, two 4 mm x 4 mm pieces were cut out from the 60MDP paper so that each piece contained the spotted cDNA or [polymerase + primer] and then placed in a PCR microtube. The tube was added with 25 µl of a PCR reaction solution (10 mM Tris-HCl, pH 8.3, 50 mM KCl, 5.3 mM MgCl, 200 µM each dNTP) and PCR was run under the following conditions:
2 min at 94°C;
29 cycles of (1 min at 94°C, 1 min at 55°C, 75 sec at 68°C); and then
15 min at 74°C.

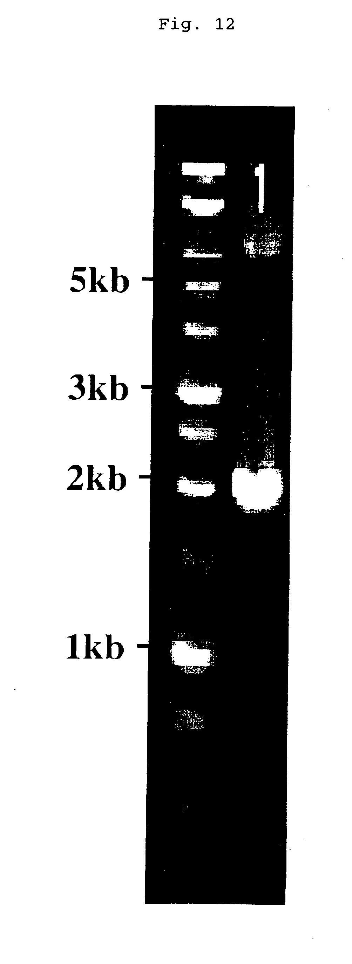

After the reaction was completed, a proper amount of the reaction solution was removed from the tube and then subjected to 1% agarose gel electrophoresis. The results are shown in Fig. 12. A band observed at the position around 1800 bp was considered to be the desired fragment. It was demonstrated that the DNA was leached out from the 60MDP paper and could be amplified by PCR.

### [Example 2]

# Experiment of [aptamer + polymerase] using lutenizing hormone gene

### <Synthesis of primers>

Primers having the following sequences were synthesized according to a conventional method.
Primer set 1 (a primer set for amplification of human lutenizing hormone gene exon 1):
HsLH1F: CCAGGGGCTGCTGCTGTTG (SEQ ID NO:4)
HsLH1R: CATGGTGGGGCAGTAGCC (SEQ ID NO:5)
Primer sets 2 (a primer set for amplification of human lutenizing hormone gene exon 2):
HsLH2F: ATGCGCGTGCTGCAGGCG (SEQ ID NO:6)
HsLH2R: TGCGGATTGAGAAGCCTTTATTG (SEQ ID NO:7)

### <Synthesis of aptamer>

An oligonucleotide having the following sequence, which is a known aptamer for Taq DNA polymerase (Yun Lin, Sumedha D. Jayasena, Inhibition of Multiple Thermostable DNA Polymerases by a Heterodimeric Aptamer, Journal of Molecular Biology (1997), Vol. 27, Issue 1, pages 100-11), was synthesized by a conventional method.
GCCGGCCAATGTACAGTATTGGCCGGC (SEQ ID NO:8)

### <Preparation of [primer + aptamer + polymerase] solution>

Two kinds of spotting solutions were prepared.

The above primer sets, the aptamer for Taq DNA polymerase and Taq DNA polymerase were mixed together to prepare a solution having the final concentrations of 2 µM of the primer sets, 2 µM of the aptamer for Taq DNA polymerase, 25 U/µl of Taq DNA polymerase and 0.1 M trehalose. Another solution having the same composition except that the aptamer for Taq DNA polymerase was eliminated was also prepared.

### <Spotting of DNA>

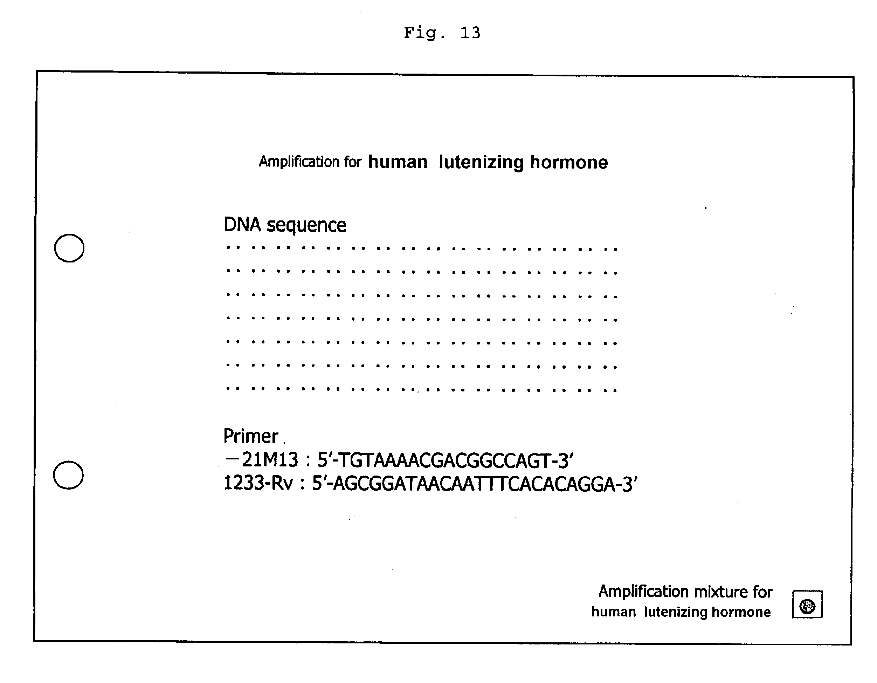

Each of the [primer + aptamer + polymerase] solutions prepared as described above was spotted on a sheet of 60MDP paper (Mishima Paper Co. , Ltd., Japan) using a 96 pin-tool (Multi 96-multiblot replicator VP409, Bio Medical Science Inc., US) so that the position and the type of the spot could be identified, as shown in Fig. 13. The spotting solution was applied at a rate of 1 µl/spot.

### <Amplification of DNA>

The spotted paper was dried at room temperature for at least 30 minutes. Thereafter, a 4 mm x 4 mm piece was cut out from the 60MDP paper so that the piece contained the spotted area and then placed in a PCR microtube. The tube was added with 25 µl of a PCR reaction solution (10 mM Tris-HCl, pH 8.3, 50 mM KCl, 5.3 mM MgCl, 200 µM each dNTP) and 50 ng of template DNA (human genomic DNA; BD Biosciences Clontech, US), and PCR was run under the following conditions:
3 min at 94°C;
50 cycles of (30 sec at 94°C, 30 sec at 40°C, 30 sec at 72°C); and then

15 min at 72°C.

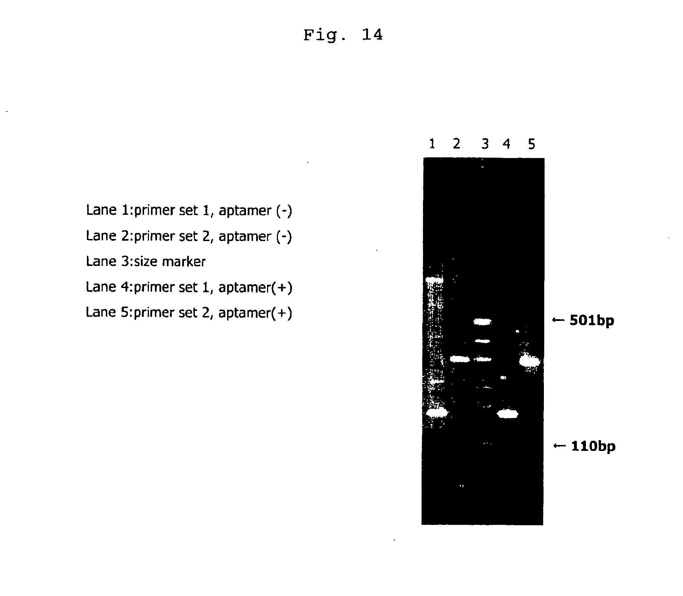

After the reaction was completed, a proper amount of the reaction solution was removed from the tube and then subjected to 1% agarose gel electrophoresis. The results are shown in Fig. 14. Bands observed at the positions around 184 bp and 343 bp were considered to be the desired DNA fragments of exons 1 and 2, respectively. It was demonstrated that fragments of interest could be amplified by PCR from the template DNA by using the primers fixed on the 60MDP paper. Comparison was made between the samples with and without the aptamer for Taq DNA polymerase, and it was found that the reaction with the aptamer could inhibit non-specific amplification.

### [Example 3]

DMA book having spots of Riken cDNA clone + PCR solution thereon

### <Synthesis of primers>

A set of primers having the following sequences were synthesized according to a conventional method.
Primer set 1:
-21M13: 5'-TGTAAAACGACGGCCAGT-3' (SEQ ID: NO:1)
1233-Rv: 5'-AGCGGATAACAATTTCACACAGGA-3' (SEQ ID NO:2)

### <Preparation of cDNA solution>

pFLC vector, into which any one of murine malate dehydrogenase cDNA (Clone ID: 1500012M15, 1758 bp), murine isocitrate dehydrogenase (NADP) (Clone ID: 1500012E04, 2440 bp), murine isocitrate dehydrogenase (NAD) (Clone ID: E030024J03, 2160 bp) and murine oxoglutarate dehydrogenase (Clone ID: E430020N12, 3554 bp) having the following nucleotide sequences collected from the Riken clones (http://fantom.gsc.riken.go.jp/) had been cloned (Fig. 10), was dissolved in TE buffer (10 mM Tris-HCl, pH 8.0, 1 mM EDTA) to give a concentration of 0.1 µg/µl.

### Murine malate dehydrogenase 1500012M15

1 cccggttctc tcccagagtc tgttccgctg tagaggtgac ctgactgctg gagactgcct 61 tttgcaggtg cagagatcgg ccttgcagtt tgcaataatg tctgaaccaa tcagagtcct
121 tgtgactgga gcagctggtc aaattgcata ttcactgttg tacagtattg gaaatggatc
181 tgtctttggg aaagaccagc ccatcattct tgtgctgttg gacatcaccc ccatgatggg
241 tgttctggac ggtgtcctga tggaactgca agactgtgcc cttccccttc tgcaggatgt
301 cattgcaacg gacaaagaag agattgcctt caaagacctg gatgtggctg tcctagtggg
361 ctccatgcca ataagggaag gcatggagag gaaggaccta ctgaaagcca atgtgaaaat
421 cttcaaatcc cagggcacag ccttggagaa atacgccaag aaatcagtta aggtcattgt
481 tgtgggaaac ccagccaata cgaactgcct gacagcctcc aagtcagcgc catcgatccc
541 caaggagaat ttcagttgcc tgactcgctt ggaccacaac cgagcaaaat ctcaaattgc
601 tcttaaactc ggtgtaaccg ctgatgatgt aaagaatgtc attatctggg gaaatcattc
661 atcgacccag tatccagatg tcaatcatgc caaggtgaaa ctgcaaggaa aggaagtcgg
721 tgtgtatgaa gccctgaaag acgacagctg gctgaaggga gagttcatca cgactgtgca
781 acagcgtggt gctgctgtca tcaaggctcg gaagctgtcc agtgcaatgt ctgctgcgaa
841 agccatcgca gaccacatca gagacatctg gtttggaacc ccagagggag agttcgtgtc
901 gatgggtgtt atctctgatg gcaactccta tggtgtccct gatgacctgc tctactcatt
961 ccctgtcgtg atcaagaata agacctggaa gtttgttgaa ggcctcccca ttaatgactt
1021 ctcccgtgaa aagatggacc tgacagcaaa ggagctgacc gaggaaaagg agaccgcttt
1081 tgagtttctc tcctctgcgt gactagacac tcgttttgac atcagcagac agccgaaggc
1141 tgaggaatca aaatgtcgtc tttgagccta gtaccaaaca gtaataatgc tacattcaaa
1201 ttgtgaacag caaaatattt taaatagtgt gtgctttatg atttgtgaaa gtctatcatg
1261 ttgttagtgc tgcaatctaa ataaaagtat attcaagtga aaatctctca gactctgttt
1321 ctactttata tttagtatct tcaggaaaac aagtttgccc aatagattat aattttactt
1381 ttttaattga ctaaaagaaa taaagatgga aaatattatg aagtaaagca ttagtctcta
1441 acataaacaa ggaagcccaa tcaatttcag agggatccca ttacttaagt ccttaaaggt
1501 tggttcatgt tttgctcata atttgatttt aaaattagct gtaagaaggt tgcagataat
1561 ctatcttctt tatattctat agcagaataa tgaagtcatt aatatttgat agccaataat
1621 accacactat taatatttgt aagctaagat tattagaaac ataaaactgt ttttgagtca
1681 gtctgttttc catgagaaga catgcatcat ctttgtgtgt tttgtgcatt actcagtgca
1741 ataaataacc ataatctc (SEQ ID NO:3)

### Murine isocitrate dehydrogenase (NADP) 1500012E04

1 gggtgttgcc gctgtcgccg cggtgaggga agtggacgcg atggccgggt ccgcgtgggt 61 gtccaaggtc tctcggctgc tgggtgcatt ccacaacaca aaacaggtga caagaggttt
121 tgctggtggt gttcagacag taactttaat tcctggagat ggaattggcc cagaaatttc
181 agcctcagtc atgaagattt ttgatgctgg ccaaagcacc tattcagtgg gaggagcgca
241 atgtcacagc aattcaagga ccaggaggaa agtgggatga tccctccaga agccaaggag
301 tccatggata agaacaagat gggcttgaaa ggcccactaa agaccccaat agccgctggc
361 catccatcta tgaatctgtt gcttcgtaag acatttgacc tttatgccaa tgtccggcca
421 tgtgtctcaa ttgaaggtta taaaacccct tacacggatg taaatatcgt caccatccga
481 gagaacacgg aaggagaata cagtggaatt gagcatgtga tcgttgatgg ggttgtgcag
541 agcatcaagc tcatcaccga agaagcaagc aagcgcattg cagagtttgc cttcgagtac
601 gctcggaaca accaccggag caacgtcaca gctgtgcaca aagctaacat catgaggatg
661 tcagatgggc tctttctgca aaaatgcagg gaagttgcgg agaactgtaa agacattaaa
721 tttaacgaga tgtaccttga tactgtatgt ttaaatatgg tacaagaccc atcccagttt
781 gatgttcttg tcatgccaaa tttatacgga gacatcctta gtgatctgtg tgcaggactg
841 attggaggtc ttggggtgac tccaagtggc aatattggag ccaacggtgt tgccatcttt
901 gaatcggttc atggaacagc cccggacatt gcaggcaagg acatggccaa ccccacggcc
961 ctcctgctta gtgctgtgat gatgcttcgc cacatgggac tttttgacca tgcagcaaaa
1021 atcgaggctg catgttttgc tacaattaag gatggaaaga gcttaacaaa agatctggga
1081 ggcaacgcga agtgctctga cttcacagaa gaaatctgtc gtagagtcaa agacttagat
1141 tagcactcct gctggtggat ttgctgcagt cagtcaatca ctccaaaagg ataccctgta
1201 atcctccttg agggcgccca ccattggttt gcttgcttct tgacagagta cgttttttga
1261 atctggcctt ttcttaacaa aacccttgca atggatgcac atgatggccc caggccttca
1321 ttcaaagggt tttcccaagt gctggttgta tttattgtcc gtctggtaaa ccttattttg
1381 taaactgtaa gtgaactgta tcatttatca ttgttaaccc attttacact tcaggcaaaa
1441 tcattttcct caactgtaaa tattctgata cagaattaat aagagaagat atttaacttt
1501 ttaacaaaag ccctggattt ttggtttatg aaaaacaaac tgggaataaa acagggtttc
1561 aacaatcgca caagataaca ttattctaat actaatgggt acaaaagaaa tttactggga
1621 aagttcacag caaaaaactg gtatatttct taaaaatatg gaaataaagt atttgtccta
1681 tacatgaatt actattaata aaaatgtaag ctccaagaaa tccataatga atgatgtaat
1741 tttgttacta catcggtaat ccttgtcaag gccccggatg ctctctgtgt atttgattct
1801 ttggttacct tgagattcac tatttggggg gaagagcttt cagataaggg agatcactcc
1861 tcactagaca gatcgtcagc attgcgagct gtcagccatg agagccagcc actgcagatc
1921 ccctcccacg tggccacact ccagccagtg ctgcaggtga ccctggaaag gcctggctgc
1981 cccttgactt tccctaaagc aaccagtcac tgccttctgc cccagtagca cccattacag
2041 acttaattgc cgaggtggag ctgactcagc ccacgctcat acaaatcagg ccaagcgggg
2101 gcctgtgtta ccagctgctg accatcaggt tctgcccctc attcttccca cagcctctgc
2161 tccacagcat gaacctagcc tttggcccac accaaagcca agctgtcttc ccttagccct
2221 tgcactagtt tgcaaactcg tggctttgca taatgtaccc tggtcccaag gggatttctt
2281 aacaacagat gtccctgtct gggtcatttt tttaaagctt ttatttggac ttacaatctt
2341 ctgtgtattt tactttaaaa ctgctgcttt ccctgtctca ctggattgtt ctggttagca
2401 gtggctttgg gttcacagta ataaagaact taagaact (SEQ ID NO:9)

### Murine isocitrate dehydrogenase (NAD) E030024J03

1 ggatctaact ggggccggct tattacagct tgtgtgtacg cgcgggtgtg agccgggtta 61 ttgaagtaaa aatgtccaga aaaatccaag gaggttctgt ggtggagatg caaggagatg
121 aaatgacacg aatcatttgg gaattgatta aggaaaaact tattcttccc tatgtggaac
181 tggatctgca tagctatgat ttaggcatag agaatcgtga tgccaccaat gaccaggtca
241 ccaaagatgc tgcagaggct ataaagaaat acaacgtggg cgtcaagtgt gctaccatca
301 cccccgatga gaagagggtt gaagaattca agttgaaaca aatgtggaaa tccccaaatg
361 gcaccatccg aaacattctg ggtggcactg tcttcaggga agctattatc tgcaaaaata
421 tcccccggct agtgacaggc tgggtaaaac ccatcatcat tggccgacat gcatatgggg
481 accaatacag agcaactgat tttgttgttc ctgggcctgg aaaagtagag ataacctaca
541 caccaaaaga tggaactcag aaggtgacat acatggtaca tgactttgaa gaaggtggtg
601 gtgttgccat gggcatgtac aaccaggata agtcaattga agactttgca cacagttcct
661 tccaaatggc tctgtccaag ggctggcctt tgtatctcag caccaagaac actattctga
721 agaagtatga tgggcgtttc aaagacatct tccaggagat ctatgacaag aaatacaagt
781 cccagtttga agctcagaag atctgctatg aacacaggct catagatgac atggtggccc
841 aagctatgaa gtccgaggga ggcttcatct gggcctgtaa gaattacgat ggggatgtgc
901 agtcagactc agtcgcccaa ggttatggct cccttggcat gatgaccagt gtgctgattt
961 gtccagatgg taagacggta gaagcagagg ctgcccatgg cactgtcaca cgtcactacc
1021 gcatgtacca gaaagggcaa gagacgtcca ccaaccccat tgcttccatt tttgcctggt
1081 cccgagggtt agcccacaga gcaaagcttg ataacaatac tgagctcagc ttcttcgcaa
1141 aggctttgga agacgtctgc attgagacca ttgaggctgg ctttatgact aaggacttgg
1201 ctgcttgcat taaaggctta cccaatgtac aacgttctga ctacttgaat acatttgagt
1261 ttatggacaa acttggagaa aacttgaagg ccaaattagc tcaggcccaa actttaaggt
1321 caaacctggg cttagaatga gtctttgcgg taactaggtc cacaggttta cgtatttttt
1381 tttttttttt tagtaacact caagattaaa aacaaaaatc attttgtaat tggtttagaa
1441 gacaaagttg aacttttata tatgtttaca gtcttttttc tttttcatac agttattgcc
1501 accttaatga atgtggtggg gaaatttttt taattgtatt ttattgtgta gtagcagtgt
1561 aggaattatg ttagtacctg ttcacaatta actgtcatgt tttctcatgc tctaatgtaa
1621 atgaccaaaa tcagaagtgc tccaagggtg aacaatagct acagtatggt tccccataag
1681 gggaaaagag aaactcactt cccctgttgt ccatgagtgt gaacactggg gcctttgtac
1741 gcaaatgttg tactgtgtgt gggagagcta tacagtaagc tcacataaga ctggaacaga
1801 taggatgtgt gtagctaaaa tgcatggcag acgtgtttat aaagagcatg tatgtgtcca
1861 atatactagt tatattttaa gaccactgga gaattccaag tctagaataa atgcagactg
1921 gaggattctg ctctttgatt tctcttctcc tgtgacccag cctaagtatt atcctacccc
1981 aagcagtaca tttcacccat gggcaataat gggagctgta ccgtttggat ttctgctgac
2041 ctgctgcatt tcttttatat aaatgtgact tttttttccc agaagttgat attaaacact
2101 attccagtct agtccttcta aactgttaat tttaattaaa atgaagtact aatgactctt
(SEQ ID NO:10)

### Murine oxoglutarate dehydrogenase E430020N12

1 gggggtggag ctgaacggga gacaggtact tgtggaaggc ttcaggacaa aatgtttcat 61 ttaaggactt gtgctgctaa gttaaggcca ttgacagcct cccagactgt taagacattt
121 tcacaaaaca aaccagcagc aattaggacg tttcaacaga ttcggtgcta ttctgcacct
181 gtagctgctg aaccatttct tagtgggact agttcgaact atgtggagga aatgtactgt
241 gcctggttgg agaatcccaa aagtgtacat aagtcatggg acattttttt ccgaaacacc
301 aatgctggag ccccaccggg cactgcctac cagagccccc tttccctgag tcgaagctcc
361 ctggctacca tggcccatgc acagtccctg gtggaagcac aacctaacgt cgacaaactc
421 gtggaggacc acttggcggt gcagtctctc atcagggcat atcagatacg agggcaccat
481 gtagcacagc tggaccccct ggggattttg gatgctgatc tggactcctc cgtgcccgct
541 gacattatct catccacaga caaacttggg ttctatggcc tacacgagtc tgaccttgac
601 aaggtcttcc acttacccac caccactttc atcgggggac aggagccagc acttcctctt
661 cgggagatca tccgtcggct ggagatggcc tactgccagc acattggtgt ggagttcatg
721 ttcattaatg atttggaaca atgccagtgg atccgacaga agtttgagac ccctggaatc
781 atgcagttca ccaatgagga gaagcggacc ttgctggcca ggcttgtacg atccaccagg
841 tttgaggagt tcctacagcg aaagtggtcc tcggagaagc gttttggtct ggaaggctgt
901 gaggtgctga tccctgccct caagacaatc attgatatgt caactcagat gaccctgaag
961 ctgtcatgta tgtatgcaag gtggcagctg agtggagaaa caccttccac aaggatgttg
1021 tagttgatct ggtgtgttat cgacgaaatg gccacaatga gatggacgaa cctatgttta
1081 cacagccact catgtacaag cagatccgca agcagaagcc tgtactgcag aagtatgcag
1141 aattgctagt ctcccagggt gtcgtcaatc agcctgagta cgaggaggaa atctccaagt
1201 atgataagat ctgtgaggaa gcatttacca gatccaaaga tgagaagatc ttgcacatca
1261 agcactggct ggattccccc tggcctggct ttttcaccct ggatggacag cccaggagca
1321 tgacctgccc ctccactggc ctggaggagg atgtcttgtt ccacattgga aaggtggcca
1381 gctctgtacc tgtggagaac tttactatcc atggagggct gagccggatc ttgaagaccc
1441 gcagagagct tgtgacgaac cggactgtgg actgggccct ggcagagtac atggcatttg
1501 gctcactgct gaaggaaggc atccatgtgc ggctgagtgg ccaggatgtg gagcggggca
1561 ccttcagcca tcgccaccat gtgctccatg atcagaatgt tgacaaaaga acctgcatcc
1621 ccatgaacca cctttggcca aatcaggccc cttacactgt atgcaacagc tcgctgtctg
1681 agtacggtgt cctgggcttt gagctgggct ttgccatggc tagccctaat gctctggttc
1741 tctgggaggc ccagtttggt gacttcaaca acatggcaca gtgcatcatt gaccagttca
1801 tctgcccagg acaggcaaag tgggtgcggc agaatggcat tgtgctcctg ctgcctcatg
1861 gcatggaagg catgggtccc gagcattcct ctgaccgccc agagcggttt ctgcatatgt
1921 gcaatgatga cccatatgtc ctgcgtgact tgcaggaaga actctttgac atcaatcagc
1981 tatatgactg caactggatt gttgtcagct gttccacccg tggcaacttc ttccatgtgc
2041 tgcgacaaca gatcttgctg cccttccgta agccgttaat agtcttcact cccaaatccc
2101 tcttgcgcca ccgtgaggca agaactatct ttgacgatat gttgccagga acgcacttcc
2161 agcgtgtgat cccagaaaat ggacatgcag ctcaggaccc tcacaaagtc aagagacttc
2221 tcttctgcac tgggaaggtg tactatgacc tcacccgaga gcgcaaagcc aggaacatga
2281 aggaggaggt ggctattaca aggattgagc agctatcacc attccccttt gacctcctgt
2341 tgaaagaggc tcagaagtat cccaatgctg agctggcctg gtgccaggaa gagcacaaga
2401 accaaggcta ctatgactat gtcaagccaa gacttcgtac caccattgac cgtgctaagc
2461 ctgtctggta tgctgtccga gacccggcag ctgctccagc cactggcaac aagaaaacac
2521 acctgacaga gctgcagcgc tttctggaca cagcctttga cctggacgca ttcaagaaat
2581 tctcttagat gctcctggag ttgatgaggc catggccccc atgtccatga cgctctttgc
2641 ttctcaacta aagaatagtg cctcagcact gtccacacgt cccttcgctg tgccacacca
2701 cccctgttct cataggaatt aagttgtcca ctgcagtgct cagctgctcc ccggtcacat
2761 gctgcccagc ctgtgccgac ttctctcagg ctgcacaccg ttcatggaga ccggaaggag
2821 cagaataagg aaagggcccc tctcaggaca tcctagagaa ggaaggcagc tctggcccca
2881 cccatgcccc cagtgcaatc ctccagggta ggaacagaac cctatgtggc ttcccagggt
2941 actagcactc agccctcgtc acccatcaag tcgcagattc aaggccagga gtagtttcat
3001 cttgctaggg ccaagctgag agctcatgga ggaactatag ctgccaggat ttgggagtca
3061 tcaggatgtt gtgtgaatag agattgtcat ggggtattta gaggacttta gcagtgatgt
3121 tagtctagcc ctgctaccct tcttgggttt gggctgtatg tgggaaactt accccagcta
3181 ccacgcctgg agagcttggc tctgagtacg gcccagaagc tccattggct cccaacgcca
3241 ggcactgctg cctcttggtc ctgctgcctc tgctctcctg acccctcccc agtcacttca
3301 ttttctctgt tgttcccttg aacacacaga agctgttgac gaattctttt ttttgctgtg
3361 ccaaggcagg tcaaaagcag atcagtggat aagagcaagt tgtcccaagg agccagctgt
3421 ccttcctccc tcttttgacc tccactggga cacacctgat ttatttattt tggttaaaaa
3481 aaaaaaggaa atgaaaaaag aacaaccacc tttgcattgc atcggcttga cccataaact
3541 aagttatcat ggtc (SEQ ID NO:11)

### <Preparation of [cDNA + primer + aptamer + polymerase + PCR buffer composition] solution>

The cDNA, the primer set 1, the aptamer for Taq DNA polymerase and Taq DNA polymerase were mixed together to prepare a solution having the final concentrations of 5 µM of the primer set 1, 5 µM of the aptamer for Taq DNA polymerase, 50 U/µl of Taq DNA polymerase, 0.1 M trehalose, 250 mM Tris-HCl (pH 8.3), 1.25 M KCl, 132.5 mM MgCl and 5 µM of each dNTP.

### <Spotting of DNA>

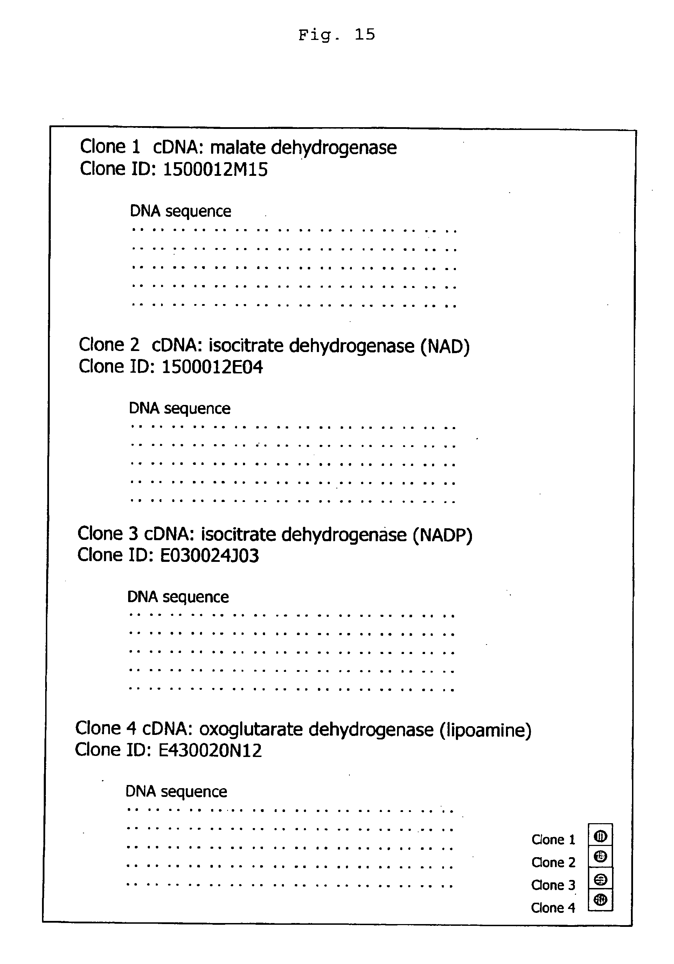

Each of the solutions prepared as described above was spotted on a sheet of 60MDP paper (Mishima Paper Co. , Ltd., Japan) using a 96 pin-tool (Multi 96-multiblot replicator VP409, Bio Medical Science Inc., US) so that the position and the type of each spot could be identified, as shown in Fig. 15. Each spotting solution was applied at a rate of 1 µl/spot.

### <Amplification of DNA>

The spotted paper was dried at room temperature for at least 30 minutes. Thereafter, four 4 mm x 4 mm pieces were cut out from the 60MDP paper so that each piece contained the spotted area and then placed in a PCR microtube. The tube was added with 25 µl of water, and PCR was run under the following conditions:
2 min at 94°C;
29 cycles of (1 min at 94°C, 1 min at 55°C, 75 sec at 68°C); and then
15 min at 74°C.

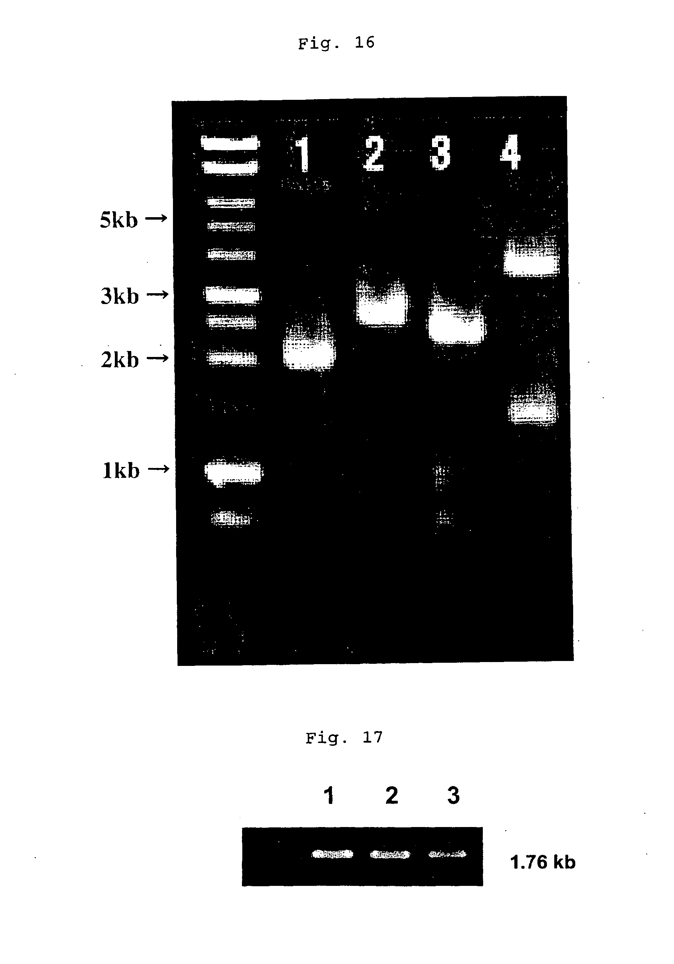

After the reaction was completed, a proper amount of the reaction solution was removed from the tube and then subjected to 1% agarose gel electrophoresis. The results are shown in Fig. 16: Lane 1, malate dehydrogenase cDNA; Lane 2, isocitrate dehydrogenase (NADP) cDNA; Lane 3, isocitrate dehydrogenase (NAD) cDNA; Lane 4, oxoglutarate dehydrogenase cDNA; and left lane, size markers. It was demonstrated that fragments having desired lengths could be obtained for the four kinds of cDNA, respectively, and that PCR amplification was possible simply by adding water to the cDNA, primers, aptamer, polymerase and PCR buffer composition fixed on the 60MDP paper.

After the reaction was completed, a proper amount of the reaction solution was removed from the tube and then subjected to 1% agarose gel electrophoresis. The results are shown in 108. Bands observed at the positions around 184 bp and 343 bp were considered to be the desired DNA fragments of exons 1 and 2, respectively. It was demonstrated that a desired fragment could be amplified by PCR from the template DNA by using the primers fixed on the 60MDP paper. Comparison was made between the samples with and without the aptamer for Taq DNA polymerase, and it was found that the reaction with the aptamer could inhibit the non-specific amplification.

### [Example 4]

### <Preparation of cDNA solution>

pFLC vector, into which the same murine malate dehydrogenase cDNA clone (Clone ID: 1500012M15, 1758 bp) as used in Example 3 had been cloned (Fig. 10), was dissolved in TE buffer (10 mM Tris-HCl, pH 8.0, 1 mM EDTA) to give a concentration of 0.1 µg/µl.

### <Preparation of [cDNA + primer + reaction enhancer (spermidine) + polymerase + PCR buffer composition] solution>

The cDNA, the primer set 1, spermidine and Taq DNA polymerase were mixed together to prepare a solution having the final concentrations of 0.005 µg/µl of the cDNA, 5 µM of the primer set, 50 U/µl of Taq DNA polymerase, 100 mM of spermidine, 0.1 M of trehalose, 250 mM of Tris-HCl (pH 8.3), 1.25 M of KCl, 132.5 mM of MgCl and 5 µM of each dNTP.

### <Spotting of DNA>

The solution prepared as described above was spotted on a sheet of 60MDP paper (Mishima Paper Co., Ltd., Japan) using a 96 pin-tool (Multi 96-multiblot replicator VP409, Bio Medical Science Inc., US) so that the position and the type of the spot could be identified, as shown in Fig. 15. The spotting solution was applied at a rate of 1 µl/spot.

### <Amplification of DNA>

The spotted paper was dried at room temperature for at least 30 minutes. Thereafter, a 4 mm x 4 mm piece was cut out from the 60MDP paper so that the piece contained the spotted area and then placed in a PCR microtube. The tube was added with 25 µl of water, and PCR was run under the following conditions:
2 min at 94°C;
29 cycles of (1 min at 94°C, 1 min at 55°C, 75 sec at 68°C); and then
15 min at 74°C.

After the reaction was completed, a proper amount of the reaction solution was removed from the tube and then subjected to 1% agarose gel electrophoresis. The results are shown in Fig. 17: Lanes 1 and 2, samples in which cDNA, primers, polymerase, spermidine (a polyamine) as reaction enhancer and PCR buffer composition were fixed on 60MDP paper; Lane 3, a sample fixing the same solution as those of Lanes 1 and 2 except that spermidine was eliminated. It was demonstrated that for each of the samples, the fixed DNA could be amplified by adding water and that in the samples on which a reaction enhancer was also fixed, the amplification reaction was enhanced.

All publications, patents and patent applications cited herein are incorporated herein by reference in their entirety.

### Industrial Applicability

The support according to the present invention can be used for storage and distribution of enzymes. The support is also applicable to printed materials and reagent kits.

### Free Text of Sequence Listing

SEQ ID NO:1 shows the nucleotide sequence of primer -21M13.
SEQ ID NO:2 shows the nucleotide sequence of primer 1233-Rv.
SEQ ID NO:3 shows the nucleotide sequence of cDNA for murine malate dehydrogenase.
SEQ ID NO:4 shows the nucleotide sequence of primer HsLH1F.
SEQ ID NO:5 shows the nucleotide sequence of primer HsLH1R.
SEQ ID NO:6 shows the nucleotide sequence of primer HsLH2F.
SEQ ID NO:7 shows the nucleotide sequence of primer HsLH2R.
SEQ ID NO:8 shows the nucleotide sequence of an aptamer for Taq DNA polymerase.
SEQ ID NO:9 shows the nucleotide sequence of cDNA for murine isocitrate dehydrogenase (NADP).
SEQ ID NO:10 shows the nucleotide sequence of cDNA for murine isocitrate dehydrogenase (NAD).
SEQ ID NO:11 shows the nucleotide sequence of cDNA for murine oxoglutarate dehydrogenase.

## Claims

1. A support comprising an enzyme and a protecting agent for the enzyme fixed thereon.

2. The support according to Claim 1, wherein the protecting agent is at least one chemical compound selected from the group consisting of trehalose and derivatives thereof.

3. The support according to Claim 1 or 2, further comprising an enhancer for enzymatic reaction.

4. The support according to any one of Claims 1 to 3, further comprising an aptamer for the enzyme.

5. A support comprising an enzyme and an aptamer for the enzyme fixed thereon.

6. The support according to any one of Claims 1 to 5, wherein the enzyme is a DNA polymerase.

7. The support according to Claim 6, further comprising a primer for the amplification of a nucleic acid of interest by a nucleic acid amplification reaction using the DNA polymerase.

8. The support according to Claim 6, further comprising at least one member selected from the group consisting of a nucleic acid which serves as a template for the nucleic acid amplification reaction using the DNA polymerase, a primer for the amplification of the nucleic acid, and a buffer for the nucleic acid amplification reaction.

9. A printed material comprising a support as recited in any one of Claims 1 to 8.

10. A reagent kit comprising a support as recited in any one of Claims 1 to 8.

11. A method for preparation of a support as recited in Claim 1, comprising: preparing a mixed solution of an enzyme and a protecting agent for the enzyme; applying the solution onto a support; and drying the support to fix a mixture of the enzyme and the protecting agent on the support.

12. A method for restoration of an enzyme fixed on a support, comprising: immersing a support as recited in any one of Claims 1 to 8 in a liquid to leach out the enzyme into the liquid.

13. A method for amplification of a nucleic acid, comprising: placing a support as recited in any one of Claims 6 to 8 in a liquid to leach out a DNA polymerase from the support; and performing a nucleic acid amplification reaction using the DNA polymerase.
